# EUROPÄISCHE PATENTSCHRIFT

(11) **EP 2 675 868 B1**
(45) Veröffentlichungstag und Bekanntmachung des Hinweises auf die Patenterteilung: **25.11.2015**
(21) Anmeldenummer: 12701829.9
(22) Anmeldetag: 18.01.2012
(51) Int. Cl.: C09K 11/06, C07D 491/00, H01L 51/00, H01L 51/50, C07D 487/14, C07F 9/6561, C07D 471/22, C07D 487/04, C07D 491/14, C07D 495/14, C07D 498/14, C07D 513/14, C07D 519/00, H01L 51/05, H01L 51/42, H05B 33/14

(54) **VERBINDUNGEN FÜR ELEKTRONISCHE VORRICHTUNGEN**
COMPOUNDS FOR ELECTRONIC DEVICES
COMPOSÉS POUR DISPOSITIFS ÉLECTRONIQUES

(30) Priorität: 17.02.2011 DE 102011011539
(43) Veröffentlichungstag der Anmeldung: 25.12.2013
(73) Patentinhaber: Merck Patent GmbH, 64293 Darmstadt (DE)
(72) Erfinder: MARTYNOVA, Irina, 64347 Griesheim (DE); PARHAM, Amir, Hossain, 65929 Frankfurt am Main (DE); PFLUMM, Christof, 64291 Darmstadt (DE)
(86) Internationale Anmeldenummer: PCT/EP2012/000206
(87) Internationale Veröffentlichungsnummer: WO 2012/110182

(56) Entgegenhaltungen:
- WO-A1-2011/157346
- JP-A- 2000 268 962
- W. V. MILLER ET AL: "Zur Synthese von Indenderivaten", BERICHTE DER DEUTSCHEN CHEMISCHEN GESELLSCHAFT, Bd. 23, 1. Januar 1890 (1890-01-01), Seiten 1881-1886, XP055025656, DOI: 10.1002/cber.18900230227

## Beschreibung

Die vorliegende Erfindung betrifft eine organische Elektrolumineszenzvorrichtung, enthaltend eine Verbindung der Formel (I), (II) und (III), oder ein Polymer, Oligomer oder Dendrimer, enthaltend eine oder mehrere Verbindungen der Formel (I), (II) oder (III). Weiterhin betrifft die Erfindung ein Verfahren zur Herstellung einer Verbindung der Formel (I), (II) oder (III) sowie eine Formulierung enthaltend eine oder mehrere Verbindungen der Formel (I), (II) oder (III).

Organische Halbleitermaterialien wie die erfindungsgemäßen Verbindungen werden für eine Reihe verschiedenartiger Anwendungen in elektronischen Vorrichtungen entwickelt. Der Aufbau organischer Elektrolumineszenzvorrichtungen (OLEDs), in denen die erfindungsgemäßen Verbindungen als funktionelle Materialien eingesetzt werden können, ist beispielsweise in US 4539507, US 5151629, EP 0676461 und WO 1998/27136 beschrieben.

Betreffend die Leistungsdaten der organischen Elektrolumineszenzvorrichtungen sind, insbesondere in Hinblick auf eine breite kommerzielle Verwendung, noch weitere Verbesserungen erforderlich. Von besonderer Bedeutung sind in diesem Zusammenhang die Lebensdauer, die Effizienz und die Betriebsspannung der organischen Elektrolumineszenz-vorrichtungen sowie die realisierten Farbwerte. Insbesondere bei blau emittierenden Elektrolumineszenzvorrichtungen besteht Verbesserungspotential bezüglich der Lebensdauer der Vorrichtungen.

Zudem ist es wünschenswert, dass die Verbindungen zur Verwendung als organische Halbleitermaterialien eine hohe thermische Stabilität und eine hohe Glasübergangstemperatur aufweisen und sich unzersetzt sublimieren lassen.

Es besteht in diesem Zusammenhang unter anderem Bedarf an an alternativen Matrixmaterialien zur Verwendung in elektronischen Vorrichtungen. Insbesondere besteht Bedarf an Matrixmaterialien für phosphoreszierende Emitter, die gleichzeitig zu guter Effizienz, hoher Lebensdauer und geringer Betriebsspannung führen. Gerade die Eigenschaften der Matrixmaterialien sind häufig limitierend für die Lebensdauer und die Effizienz der organischen Elektrolumineszenzvorrichtung.

Gemäß dem Stand der Technik werden häufig Carbazolderivate, z. B. Bis(carbazolyl)biphenyl, als Matrixmaterialien verwendet. Hier besteht noch Verbesserungspotential insbesondere in Bezug auf die Lebensdauer und die Glasübergangstemperatur der Materialien.

Weiterhin werden gemäß dem Stand der Technik Ketonverbindungen, z. B. wie in WO 2005/084081, WO 2004/093207 und WO 2004/013080 beschrieben, als Matrixmaterialien in OLEDs eingesetzt. Nochmals weiterhin werden gemäß dem Stand der Technik Triazinverbindungen als Matrixmaterialien verwendet, z. B. wie in WO 2010/015306, WO 2007/063754 oder WO 2008/056746 offenbart. Es besteht jedoch weiterhin Bedarf an alternativen Verbindungen mit anderer chemischer Struktur, insbesondere solchen, die eine Verbesserung betreffend Lebensdauer, Effizienz und Betriebsspannung bewirken.

Von besonderem Interesse ist weiterhin die Bereitstellung von alternativen Materialien als Matrixkomponenten von Mixed-Matrix-Systemen. Unter einem Mixed-Matrix-System wird im Sinne dieser Anmeldung ein System verstanden, in dem zwei oder mehr verschiedene Matrixverbindungen zusammen mit einer (alternativ auch mehreren) Dotandverbindungen gemischt als emittierende Schicht verwendet werden. Diese Systeme sind insbesondere von Interesse bei phosphoreszierenden organischen Elektrolumineszenzvorrichtungen. Für detailliertere Informationen wird auf die Anmeldung WO 2010/108579 verwiesen. Als im Stand der Technik bekannte Verbindungen als Matrixkomponenten in Mixed-Matrix-Systemen sind unter anderem CBP (Biscarbazolylbiphenyl) und TCTA (Triscarbazolyltriphenylamin) zu nennen. Es besteht jedoch weiterhin Bedarf an alternativen Verbindungen zur Verwendung als Matrixkomponenten in Mixed-Matrix-Systemen. Insbesondere besteht Bedarf an Verbindungen, welche eine Verbesserung der Betriebsspannung und Lebensdauer der elektronischen Vorrichtungen bewirken.

Weiterhin ist die Bereitstellung neuer Elektronentransportmaterialien wünschenswert, da gerade auch die Eigenschaften des Elektronentransportmaterials einen wesentlichen Einfluss auf die oben genannten Eigenschaften der organischen Elektrolumineszenzvorrichtung ausüben. Insbesondere besteht Bedarf an Elektronentransportmaterialien, welche gleichzeitig zu guter Effizienz, hoher Lebensdauer und geringer Betriebsspannung führen.

In den Anmeldungen WO 2010/136109 und WO 2011/000455 werden Indenocarbazol- und Indolocarbazolderivate mit unterschiedlicher Verknüpfungsgeometrie der Inden- bzw. Indol- und der Carbazoleinheit offenbart. Die Verbindungen eignen sich sehr gut zur Verwendung als Funktionsmaterialien in organischen Elektrolumineszenzvorrichtungen, insbesondere als Matrixmaterialien für phosphoreszierende Emitter sowie als Elektronentransportmaterialien. Die Synthese von Indenderivaten ist weiterhin bekannt (z. B. W.v. Miller und Ruhde, diese Berichte XXII, 1830). Es besteht jedoch weiterhin Bedarf an alternativen Verbindungen, insbesondere solchen, mit denen eine Senkung der Betriebsspannung, eine Erhöhung der Leistungseffizienz sowie eine Erhöhung der Lebensdauer erreicht werden kann.

In der Anmeldung JP 2006/066580 werden Carbazolderivate mit ankondensierten aromatischen Ringen offenbart, unter anderem zur Verwendung als Hostmaterialien in einer organischen Elektrolumineszenzvorrichtung.

In der Anmeldungen JP 2007/088016 und JP 2000/268962 werden organische Halbleitermaterialien, welche mehrere miteinander kondensierte Pyrrolringe aufweisen, unter anderem zur Verwendung in OLEDs offenbart.

In der noch nicht offengelegten Anmeldung DE 102010033548.7 werden organische Halbleitermaterialien, welche mehrere miteinander kondensierte heteroaromatische Fünfringe aufweisen, offenbart. Die Materialien werden gemäß der genannten Anmeldung bevorzugt in OLEDs verwendet.

Die vorliegende Erfindung betrifft eine organische Elektrolumineszenzvorrichtung, enthaltend eine Verbindung der Formel (I), (II) und (III), oder ein Polymer, Oligomer oder Dendrimer, enthaltend eine oder mehrere Verbindungen der Formel (I), (II) oder (III). Die vorteilhaften Eigenschaften von so einer Elektrolumineszenzvorrichtung werden in einem der folgenden Abschnitte sowie in den experimentellen Beispielen im Detail dargelegt.

Gegenstand der Erfindung ist somit eine organische Elektrolumineszenzvorrichtung, enthaltend eine Verbindung der Formel (I), (II) oder (III) 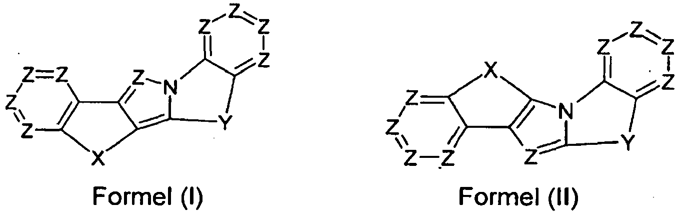 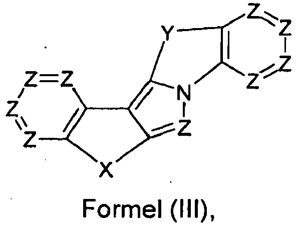 wobei für die auftretenden Symbole gilt:
- Z: ist bei jedem Auftreten gleich oder verschieden ausgewählt aus CR² und N;
- X, Y: sind bei jedem Auftreten gleich oder verschieden eine divalente Gruppe, ausgewählt aus BR², C(R²)₂, C=O, C=NR², C=S, Si(R²)₂, NR¹, PR², P(=O)R², O, S, S=O und S(=O)₂;
- R¹: ist bei jedem Auftreten gleich oder verschieden ein aromatisches oder heteroaromatisches Ringsystem mit 5 bis 60 aromatischen Ringatomen, welches mit einem oder mehreren Resten R³ substituiert sein kann und welches mit einem Substituenten R² am Grundgerüst oder einem Atom des Grundgerüsts verknüpft sein kann;
- R²: ist bei jedem Auftreten gleich oder verschieden H, D, F, Cl, Br, I, B(OR³)₂, CHO, C(=O)R³, CR³=C(R³)₂, CN, C(=O)OR³, C(=O)N(R³)₂, Si(R³)₃, N(R³)₂, N(Ar¹)₂, NO₂, P(=O)(R³)₂, OSO₂R³, OR³, S(=O)R³, S(=O)₂R³, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere benachbarte oder nicht benachbarte CH₂-Gruppen in den oben genannten Gruppen durch -R³C=CR³-, -C≡C-, Si(R³)₂, Ge(R³)₂, Sn(R³)₂, C=O, C=S, C=Se, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R³ substituiert sein kann, oder eine Aryloxy- oder Heteroaryloxygruppe mit 5 bis 60 aromatischen Ringatomen, die durch einen oder mehrere Reste R³ substituiert sein kann, wobei zwei oder mehr Reste R² miteinander verknüpft sein können und einen Ring oder ein Ringsystem bilden können;
- R³: ist bei jedem Auftreten gleich oder verschieden H, D, F, Cl, Br, I, B(OR⁴)₂, CHO, C(=O)R⁴, CR⁴=C(R⁴)₂, CN, C(=O)OR⁴, C(=O)N(R⁴)₂, Si(R⁴)₃, N(R⁴)₂, NO₂, P(=O)(R⁴)₂, OSO₂R⁴, OR⁴, S(=O)R⁴, S(=O)₂R⁴, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere benachbarte oder nicht benachbarte CH₂-Gruppen in den oben genannten Gruppen durch -R⁴C=CR⁴-, -C=C-, Si(R⁴)₂, Ge(R⁴)₂, Sn(R⁴)₂, C=O, C=S, C=Se, C=NR⁴, -C(=O)O-, -C(=O)NR⁴- , NR⁴, P(=O)(R⁴), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R⁴ substituiert sein kann, oder eine Aryloxy- oder Heteroaryloxygruppe mit 5 bis 60 aromatischen Ringatomen, die durch einen oder mehrere Reste R⁴ substituiert sein kann, wobei zwei oder mehr Reste R³ miteinander verknüpft sein können und einen Ring oder ein Ringsystem bilden können;
- R⁴: ist gleich oder verschieden bei jedem Auftreten H, D, F oder ein aliphatischer, aromatischer und/oder heteroaromatischer organischer Rest mit 1 bis 20 C-Atomen, in dem auch ein oder mehrere H-Atome durch D oder F ersetzt sein können; dabei können zwei oder mehr Substituenten R⁴ auch miteinander verknüpft sein und einen Ring oder ein Ringsystem bilden;
- Ar¹: ist bei jedem Auftreten gleich oder verschieden ein aromatisches oder heteroaromatisches Ringsystem mit 5 bis 30 aromatischen Ringatomen, das mit einem oder mehreren Resten R³ substituiert sein kann;
oder ein Polymer, Oligomer oder Dendrimer, enthaltend eine oder mehrere Verbindungen der Formel (I), (II) oder (III), wobei die Bindung(en) zum Polymer, Oligomer oder Dendrimer an beliebigen, in Formel (I), (II) bzw. (III) mit R¹ oder R² substituierten Positionen lokalisiert sein können,
wobei die Verbindung der Formel (I), (II) oder (II), oder das Polymer, Oligomer oder Dendrimer, enthaltend eine oder mehrere Verbindungen der Formel (I), (II) oder (III) als Lochtransportmaterial in einer Lochtransportschicht oder Lochinjektionsschicht eingesetzt wird und/oder als Matrixmaterial in einer
emittierenden Schicht eingesetzt wird und/oder als Elektronentransportmaterial in einer Elektrontransportschicht eingesetzt wird.

Bevorzugt sind in den erfindungsgemäßen Verbindungen 0, 1, 2, 3, 4, 5 oder 6 Gruppen Z gleich N, und die restlichen Gruppen Z sind gleich CR². Weiterhin bevorzugt sind 0, 1 oder 2 Gruppen Z pro aromatischem Sechsring in einer Verbindung der Formel (I), (II) oder (III) gleich N, und die restlichen Gruppen Z sind gleich CR².

Gemäß einer bevorzugten Ausführungsform der Erfindung ist X ausgewählt aus BR², C=O, NR¹, PR², P(=O)R², O, S, S=O und S(=O)₂. Besonders bevorzugt ist X ausgewählt aus C=O, NR¹, O, S, S=O und S(=O)₂. Ganz besonders bevorzugt ist X gleich NR¹.

Weiterhin ist gemäß einer bevorzugten Ausführungsform der Erfindung Y ausgewählt aus C(R²)₂, C=O, NR¹, PR², P(=O)R², O, S und S(=O)₂. Besonders bevorzugt ist Y ausgewählt aus C(R²)₂, NR¹, O und S. Ganz besonders bevorzugt ist Y gleich C(R²)₂.

In einer besonders bevorzugten Ausführungsform der Erfindung treten die bevorzugten Ausführungsformen von X und Y kombiniert miteinander auf. Ebenfalls bevorzugt ist das kombinierte Auftreten der besonders bevorzugten oder der ganz besonders bevorzugten Ausführungsformen von X und Y.

In einer bevorzugten Ausführungsform der Erfindung ist R¹ bei jedem Auftreten gleich oder verschieden ausgewählt aus einem aromatischen oder heteroaromatischen Ringsystem mit 5 bis 30 aromatischen Ringatomen, welches mit einem oder mehreren Resten R³ substituiert sein kann. Besonders bevorzugt umfasst das aromatische oder heteroaromatische Ringsystem eine oder mehrere Aryl- oder Heteroarylgruppen ausgewählt aus Benzol, Naphthalin, Anthracen, Phenanthren, Pyren, Chrysen, Benzanthracen, Tetracen, Pentacen, Furan, Benzofuran, Thiophen, Benzothiophen, Pyrrol, Indol, Isoindol, Carbazol, Pyridin, Chinolin, Isochinolin, Pyrazol, Imidazol, Benzimidazol, Pyridazin, Pyrimidin, Pyrazin, Triazol, Benzotriazol und Triazin, welche jeweils mit einem oder mehreren Resten R³ substituiert sein können. Die Aryl- bzw. Heteroarylgruppen sind bevorzugt durch Einfachbindungen und/oder divalente Gruppen ausgewählt aus Alkylengruppen mit 1 bis 8 Kohlenstoffatomen, Alkenylengruppen mit 2 bis 8 Kohlenstoffatomen, C=O, NR³, O oder S miteinander verbunden.

In einer weiteren bevorzugten Ausführungsform der Erfindung ist R² bei jedem Auftreten gleich oder verschieden ausgewählt aus H, D, F, CN, Si(R³)₃, N(R³)₂ oder einer geradkettigen Alkyl- oder Alkoxygruppe mit 1 bis 20 C-Atomen oder einer verzweigten oder cyclischen Alkyl- oder Alkoxygruppe mit 3 bis 20 C-Atomen, wobei die oben genannten Gruppen jeweils mit einem oder mehreren Resten R³ substituiert sein können und wobei in den oben genannten Gruppen eine oder mehrere benachbarte oder nicht benachbarte CH₂-Gruppen durch -C=C-, -R³C=CR³-, Si(R³)₂, C=O, C=NR³, -NR³-, -O-, -S-, -C(=O)O- oder -C(=O)NR³- ersetzt sein können, oder ein aromatisches oder heteroaromatisches Ringsystem mit 5 bis 20 aromatischen Ringatomen, das jeweils mit einem oder mehreren Resten R³ substituiert sein kann.

In einer weiteren bevorzugten Ausführungsform ist R³ bei jedem Auftreten gleich oder verschieden ausgewählt aus H, D, F, CN, Si(R⁴)₃, N(R⁴)₂ oder einer geradkettigen Alkyl- oder Alkoxygruppe mit 1 bis 20 C-Atomen oder einer verzweigten oder cyclischen Alkyl- oder Alkoxygruppe mit 3 bis 20 C-Atomen, wobei die oben genannten Gruppen jeweils mit einem oder mehreren Resten R⁴ substituiert sein können und wobei in den oben genannten Gruppen eine oder mehrere benachbarte oder nicht benachbarte CH₂-Gruppen durch -C≡C-, -R⁴C=CR⁴-, Si(R⁴)₂, C=O, C=NR⁴, -NR⁴-, -O-, -S-, -C(=O)O- oder -C(=O)NR⁴- ersetzt sein können, oder ein aromatisches oder heteroaromatisches Ringsystem mit 5 bis 20 aromatischen Ringatomen, das jeweils mit einem oder mehreren Resten R⁴ substituiert sein kann, wobei zwei oder mehr Reste R³ miteinander verknüpft sein können und einen Ring oder ein Ringsystem bilden können.

Es ist weiterhin bevorzugt, dass die erfindungsgemäßen Verbindungen als Substituent R¹ oder R² mindestens eine Gruppe tragen, welche ausgewählt ist aus
- Heteroarylgruppen mit 1 bis 20 C-Atomen, welche wahlweise über eine oder mehrere dazwischen gebundene divalente Arylgruppen gebunden sein können und welche mit einem oder mehreren Resten R³ substituiert sein können,
- aromatischen oder heteroaromatischen Ringsystemen mit 5 bis 20 aromatischen Ringatomen, welche mit einem oder mehreren Resten R³ substituiert sein können, und
- Arylamingruppen, welche mit einem oder mehreren Resten R³ substituiert sein können,
wobei die oben genannten Heteroarylgruppen bevorzugt ausgewählt sind aus Pyridin, Pyrimidin, Pyridazin, Pyrazin, Triazin und Benzimidazol, welche mit einem oder mehreren Resten R³ substituiert sein können und wobei die oben genannten aromatischen oder heteroaromatischen Ringsysteme bevorzugt ausgewählt sind aus Naphthyl, Anthracenyl, Phenanthrenyl, Benzanthracenyl, Pyrenyl, Biphenyl, Terphenyl und Quaterphenyl, welche mit einem oder mehreren Resten R³ substituiert sein können.

Die oben genannten Arylamingruppen stellen bevorzugt Gruppen der folgenden Formel (A) dar 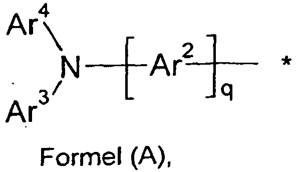 wobei das Symbol * die Bindung zum Rest der Verbindung markiert und weiterhin
- Ar², Ar³, Ar⁴: gleich oder verschieden eine Aryl- oder Heteroarylgruppe mit 5 bis 20 aromatischen Ringatomen darstellen, welche mit einem oder mehreren Resten R³ substituiert sein kann, wobei eine oder mehrere Kombinationen ausgewählt aus Ar² und Ar², Ar² und Ar³, Ar² und Ar⁴ und Ar³ und Ar⁴ jeweils durch eine Einfachbindung oder eine bivalente Gruppe ausgewählt aus BR³, C(R³)₂, C=O, C=NR³, C=C(R³)₂, C=S, Si(R³)₂, NR³, O, S, S=O und S(=O)₂ miteinander verknüpft sein können; und
- q: gleich 0, 1, 2, 3, 4 oder 5 ist.

Die oben genannten Heteroarylgruppen, welche wahlweise über eine oder mehrere dazwischen gebundene divalente Arylgruppen gebunden sein können, stellen bevorzugt Gruppen der folgenden Formel (B) dar 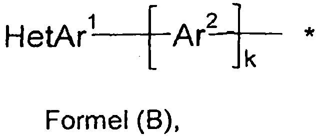 wobei das Symbol * die Bindung zum Rest der Verbindung markiert und weiterhin
- Ar²: wie oben definiert ist,
- k: gleich 0, 1, 2 oder 3 ist, und

HetAr¹ eine Heteroarylgruppe mit 1 bis 20 C-Atomen darstellt, welche mit einem oder mehreren Resten R³ substituiert sein kann.

Bevorzugte Ausführungsformen der Verbindungen gemäß Formel (I), (II) und (III), bei denen die Gruppen X und Y wie angegeben definiert sind, sind in der folgenden Tabelle aufgeführt (Formeln (I-1) bis (I-32), (II-1) bis (II-32) und (III-1) bis (III-32)).

| | X | Y |
|---|---|---|
| (I-1) | C=O | C(R²)₂ |
| (I-2) | C=O | C=O |
| (I-3) | C=O | NR¹ |
| (I-4)_{.} | C=O | PR² |
| (I-5) | C=O | PR²(=O) |
| (I-6) | C=O | O |
| (I-7) | C=O | S |
| (I-8) | C=O | S(=O)₂ |
| (I-9) | NR¹ | C(R²)₂ |
| (I-10) | NR¹ | C=O |
| (I-11) | NR¹ | NR¹ |
| (I-12) | NR¹ | PR² |
| (I-13) | NR¹ | PR²(=O) |
| (I-14) | NR¹ | O |
| (I-15) | NR¹ | S |
| (I-16) | NR¹ | S(=O)₂ |
| (I-17) | S | C(R²)₂ |
| (I-18) | S | C=O |
| (I-19) | S | NR¹ |
| (I-20) | S | PR² |
| (I-21) | S | PR²(=O) |
| (I-22) | S | O |
| (I-23) | S | S |
| (I-24) | S | S(=O)₂ |
| (I-25) | S(=O)₂ | C(R²)₂ |
| (I-26) | S(=O)₂ | C=O |
| (I-27) | S(=O)₂ | NR¹ |
| (I-28) | S(=O)₂ | PR² |
| (I-29) | S(=O)₂ | PR²(=O) |
| (I-30) | S(=O)₂ | O |
| (I-31) | S(=O)₂ | S |
| (I-32) | S(=O)₂ | S(=O)₂ |
| (II-1) | C=O | C(R²)₂ |
| (II-2) | C=O | C=O |
| (II-3) | C=O | NR¹ |
| (II-4) | C=O | PR² |
| (II-5) | C=O | PR²(=O) |
| (II6) | C=O | O |
| (II-7) | C=O | S |
| (II-8) | C=O | S(=O)₂ |
| (II-9) | NR¹ | C(R²)₂ |
| (II-10) | NR¹ | C=O |
| (II-11) | NR¹ | NR¹ |
| (II-12) | NR¹ | PR² |
| (II-13) | NR¹ | PR²(=O) |
| (II-14) | NR¹ | O |
| (II-15) | NR¹ | S |
| (II-16) | NR¹ | S(=O)₂ |
| (II-17) | S | C(R²)₂ |
| (II-18) | S | C=O |
| (II-19) | S | NR¹ |
| (II-20) | S | PR² |
| (II-21) | S | PR²(=O) |
| (II-22) | S | O |
| (II-23) | S | S |
| (II-24) | S | S(=O)₂ |
| (II-25) | S(=O)₂ | C(R²)₂ |
| (II-26) | S(=O)₂ | C=O |
| (II-27) | S(=O)₂ | NR¹ |
| (II-28) | S(=O)₂ | PR² |
| (II-29) | S(=O)₂ | PR²(=O) |
| (II-30) | S(=O)₂ | O |
| (II-31) | S(=O)₂ | S |
| (II-32) | S(=O)₂ | S(=O)₂ |
| (III-1) | C=O | C(R²)₂ |
| (III-2) | C=O | C=O |
| (III-3) | C=O | NR¹ |
| (III-4) | C=O | PR² |
| (III-5) | C=O | PR²(=O) |
| (III-6) | C=O | O |
| (III-7) | C=O | S |
| (III-8) | C=O | S(=O)₂ |
| (III-9) | NR¹ | C(R²)₂ |
| (III-10) | NR¹ | C=O |
| (III-11) | NR¹ | NR¹ |
| (III-12) | NR¹ | PR² |
| (III-13) | NR¹ | PR²(=O) |
| (III-14) | NR¹ | O |
| (III-15) | NR¹ | S |
| (III-16) | NR¹ | S(=O)₂ |
| (III-17) | S | C(R²)₂ |
| (III-18) | S | C=O |
| (III-19) | S | NR¹ |
| (III-20) | S | PR² |
| (III-21) | S | PR²(=O) |
| (III-22) | S | O |
| (III-23) | S | S |
| (III-24) | S | S(=O)₂ |
| (III-25) | S(=O)₂ | C(R²)₂ |
| (III-26) | S(=O)₂ | C=O |
| (III-27) | S(=O)₂ | NR¹ |
| (III-28) | S(=O)₂ | PR² |
| (III-29) | S(=O)₂ | PR²(=O) |
| (III-30) | S(=O)₂ | O |
| (III-31) | S(=O)₂ | S |
| (III-32) | S(=O)₂ | S(=O)₂ |

Gemäß einer besonders bevorzugten Ausführungsform der vorliegenden Erfindung treten die in der Tabelle aufgeführten bevorzugten Formeln in Kombination mit den oben angegebenen bevorzugten Ausführungsformen der Gruppen Z, R¹, R² und R³ auf.

Die folgende Tabelle zeigt Beispiele für Verbindungen gemäß der vorliegenden Erfindung.

| | | |
|---|---|---|
| 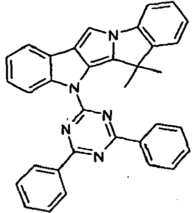 | 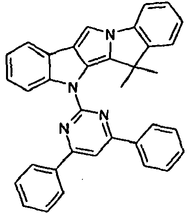 | 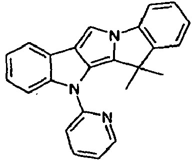 |
| 1 | 2 | 3 |
| 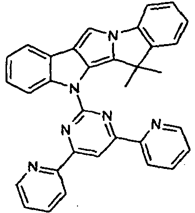 | 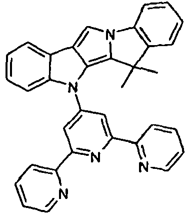 | 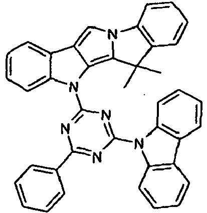 |
| 4 | 5 | 6 |
| 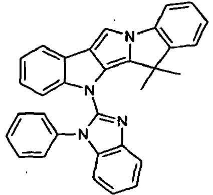 | 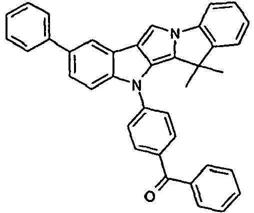 | 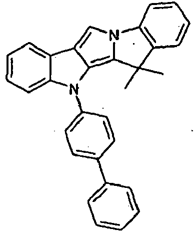 |
| 7 | 8 | 9 |
| 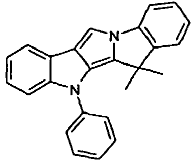 | 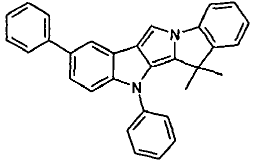 | 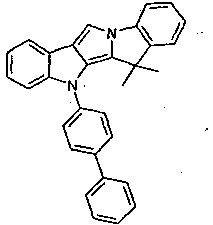 |
| 10 | 11 | 12 |
| 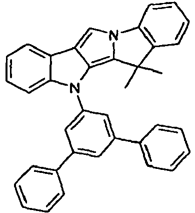 | 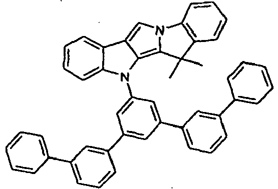 | 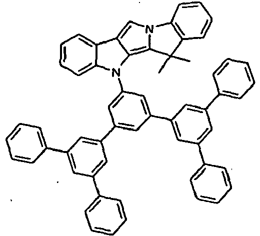 |
| 13 | 14 | 15 |
| 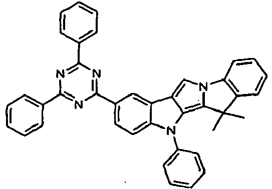 | 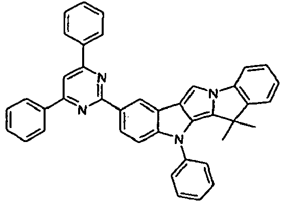 | 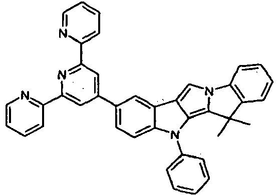 |
| 16 | 17 | 18 |
| 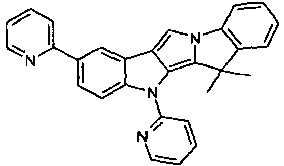 | 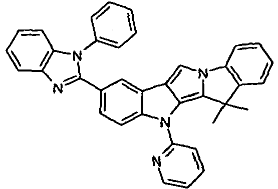 | 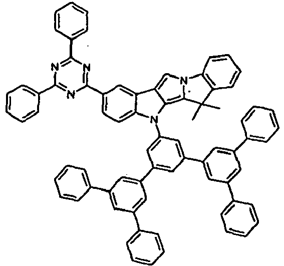 |
| 19 | 20 | 21 |
| 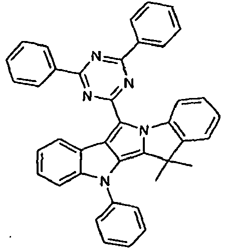 | 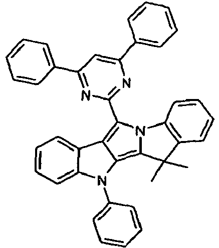 | 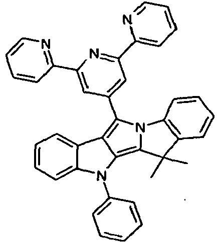 |
| 22 | 23 | 24 |
| 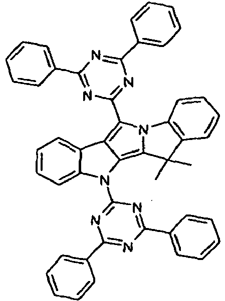 | 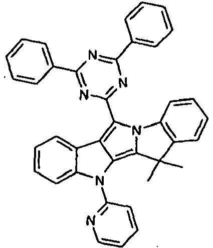 | 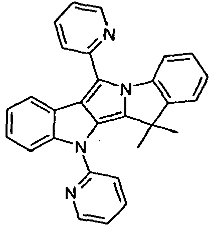 |
| 25 | 26 | 27 |
| 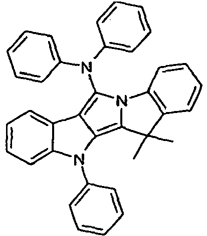 | 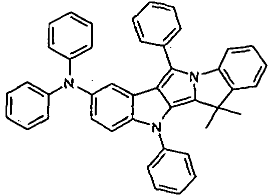 | 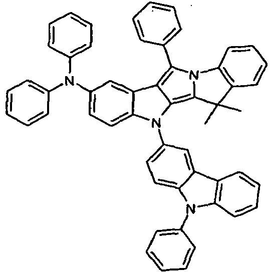 |
| 28 | 29 | 30 |
| 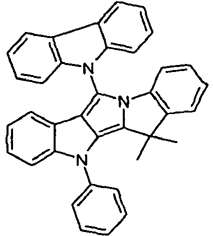 | 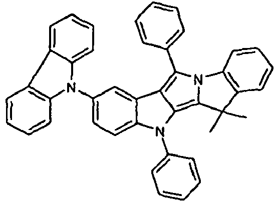 | 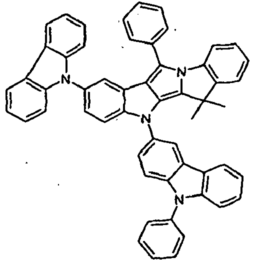 |
| 31 | 32 | 33 |
| 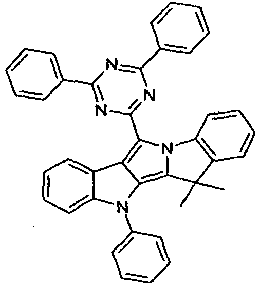 | 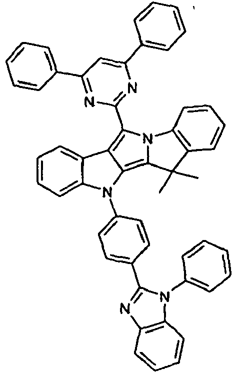 | 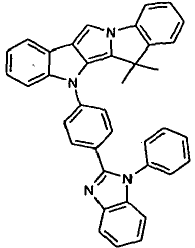 |
| 34 | 35 | 36 |
| 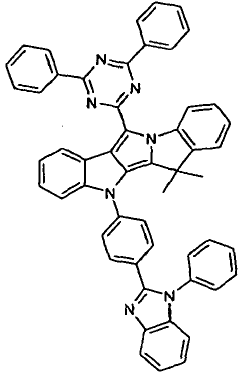 | 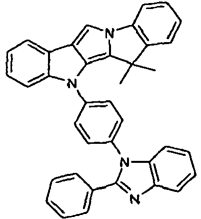 | 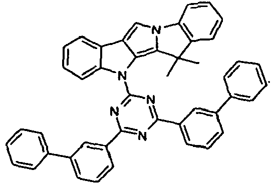 |
| 37 | 38 | 39 |
| 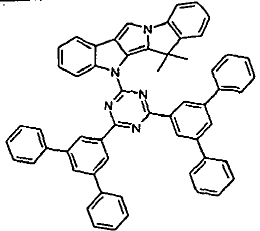 | 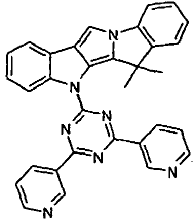 | 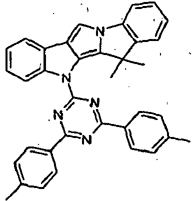 |
| 40 | 41 | 42 |
| 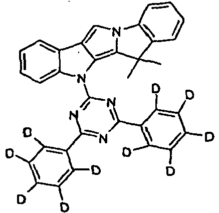 | 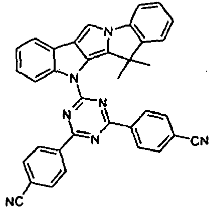 | 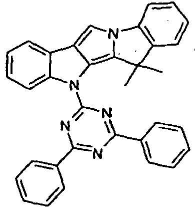 |
| 43 | 44 | 45 |
| 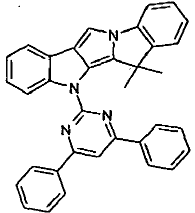 | 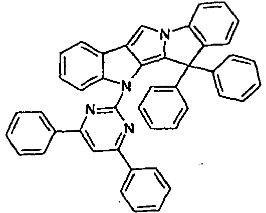 | 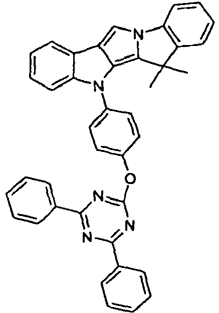 |
| 46 | 47 | 48 |
| 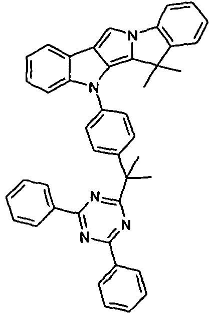 | 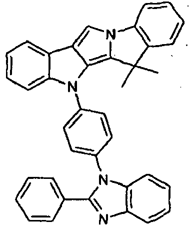 | 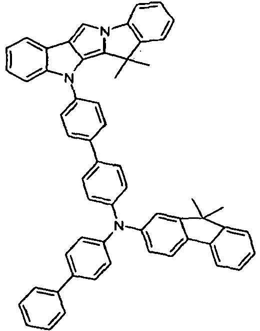 |
| 49 | 50 | 51 |
| 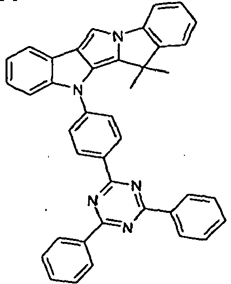 | 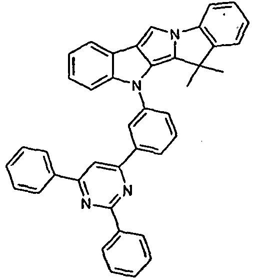 | 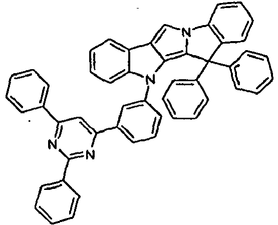 |
| 52 | 53 | 54 |
| 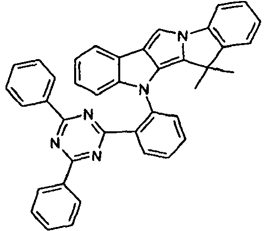 | 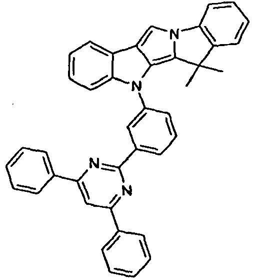 | 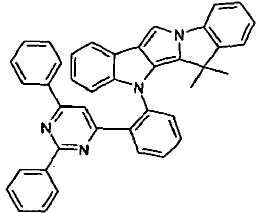 |
| 55 | 56 | 57 |
| 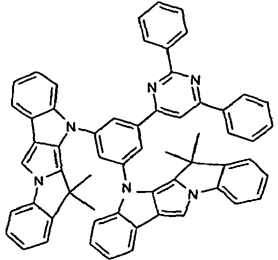 | 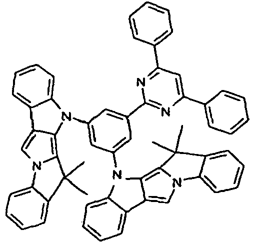 | 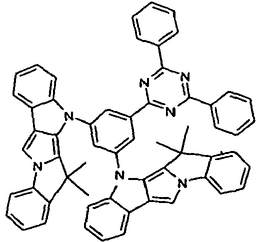 |
| 58 | 59 | 60 |
| 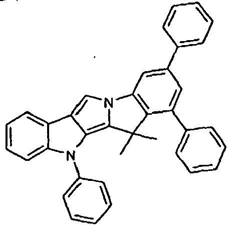 | 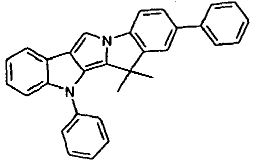 | 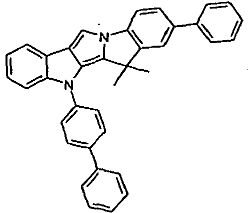 |
| 61 | 62 | 63 |
| 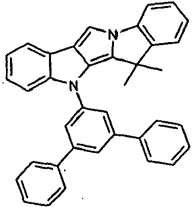 | 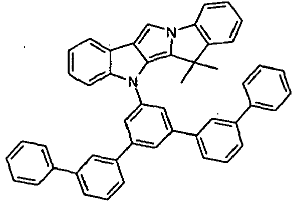 | 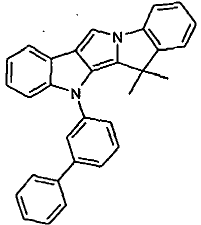 |
| 64 | 65 | 66 |
| 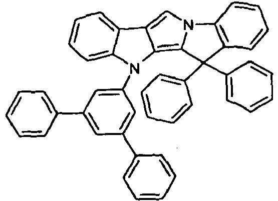 | 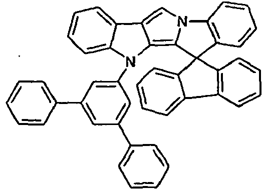 | 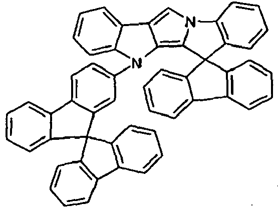 |
| 67 | 68 | 69 |
| 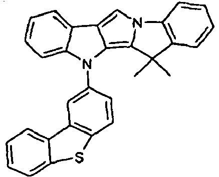 | 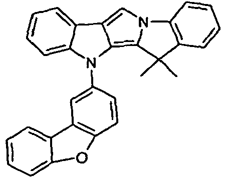 | 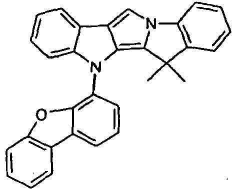 |
| 70 | 71 | 72 |
| 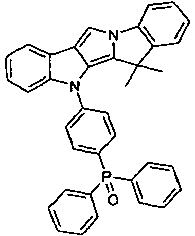 | 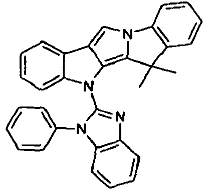 | 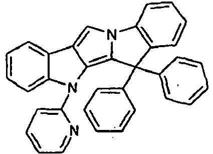 |
| 73 | 74 | 75 |
| 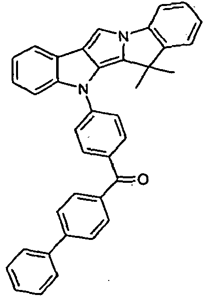 | 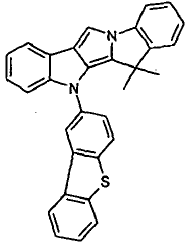 | 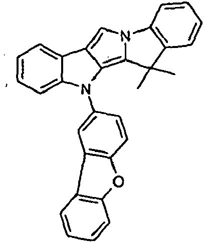 |
| 76 | 77 | 78 |
| 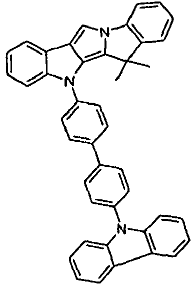 | 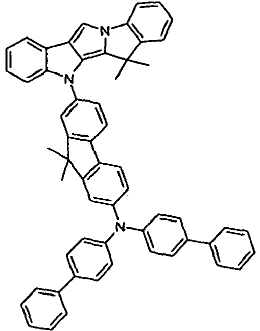 | 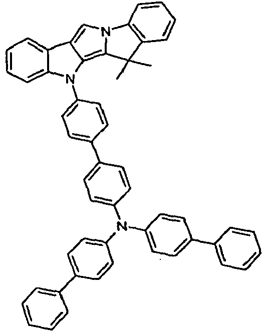 |
| 79 | 80 | 81 |
| 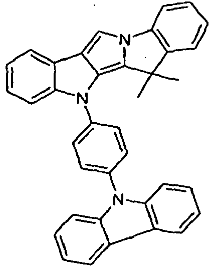 | 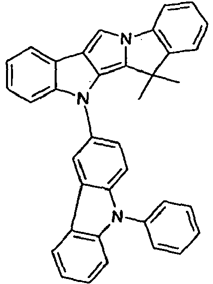 | 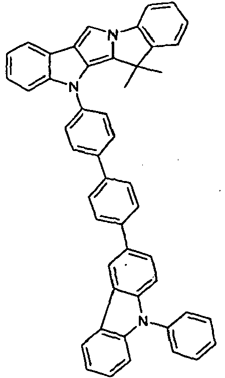 |
| 82 | 83 | 84 |
| 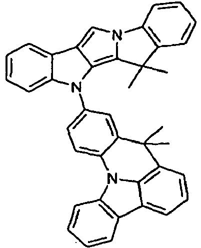 | 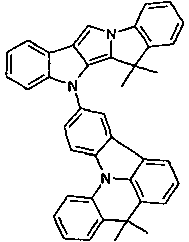 | 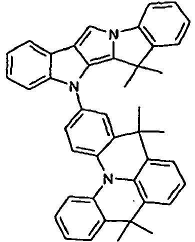 |
| 85 | 86 | 87 |
| 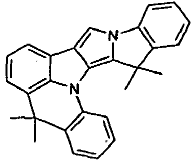 | 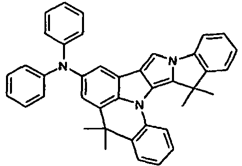 | 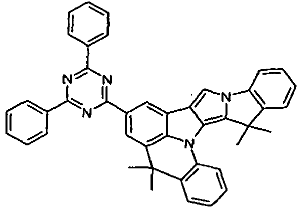 |
| 88 | 89 | 90 |
| 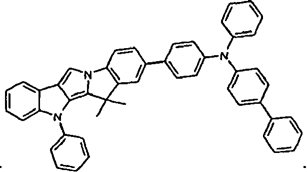 | 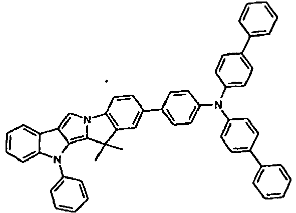 | 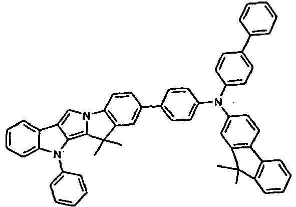 |
| 91 | 92 | 93 |
| 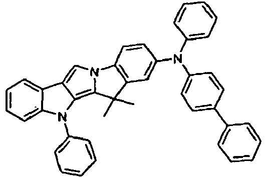 | 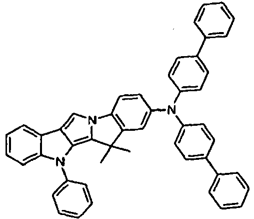 | 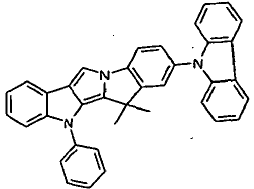 |
| 94 | 95 | 96 |
| 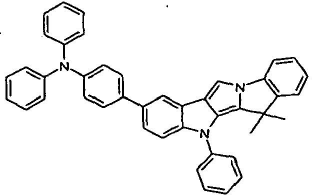 | 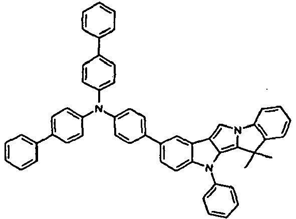 | 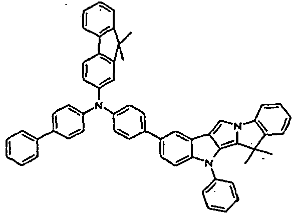 |
| 97 | 98 | 99 |
| 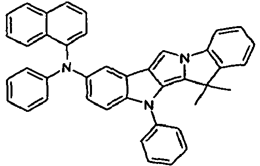 | 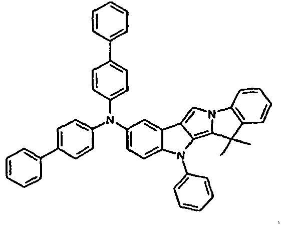 | 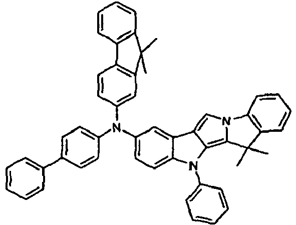 |
| 100 | 101 | 102 |
| 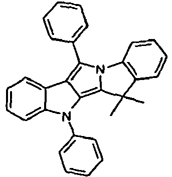 | 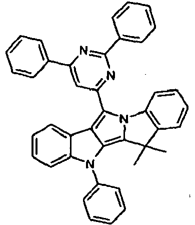 | 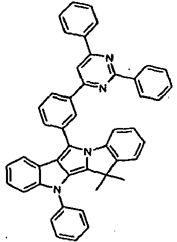 |
| 103 | 104 | 105 |
| 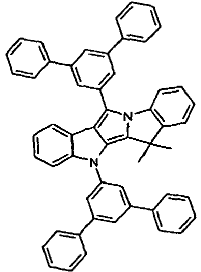 | 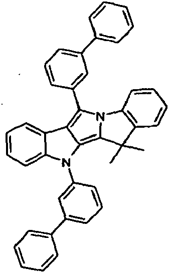 | 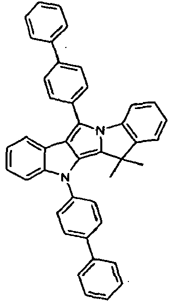 |
| 106 | 107 | 108 |
| 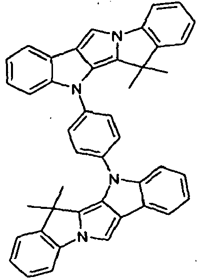 | 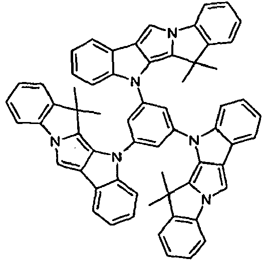 | 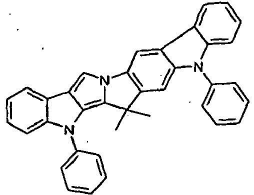 |
| 109 | 110 | 111 |
| 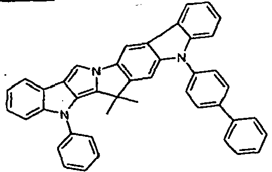 | 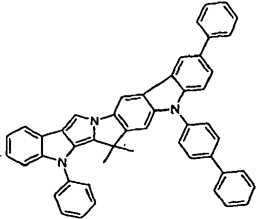 | 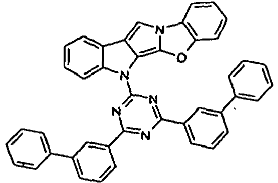 |
| 112 | 113 | 114 |
| 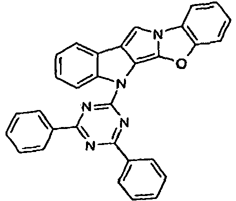 | 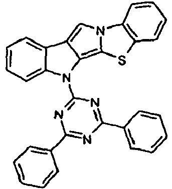 | 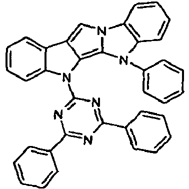 |
| 115 | 116 | 117 |
| 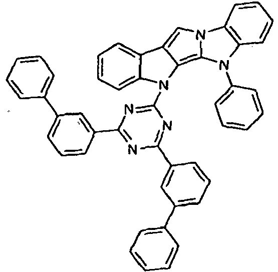 | 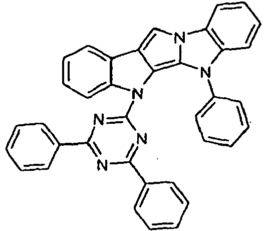 | 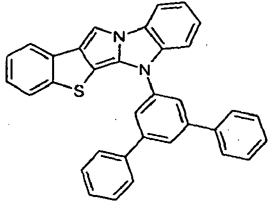 |
| 118 | 119 | 120 |
| 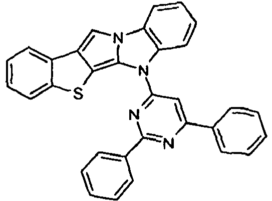 | 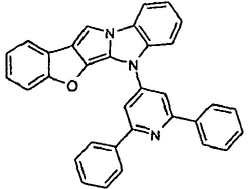 | 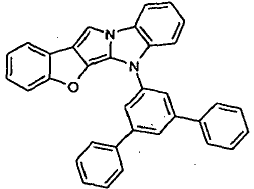 |
| 121 | 122 | 123 |
| 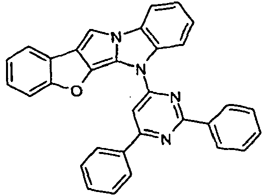 | 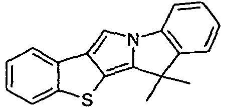 | 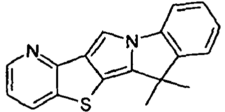 |
| 124 | 125 | 126 |
| 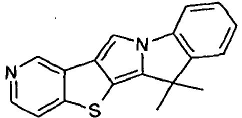 | 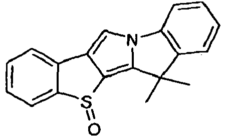 | 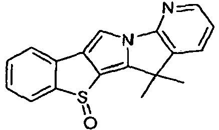 |
| 127 | 128 | 129 |
| 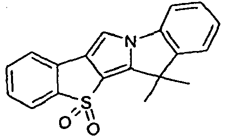 | 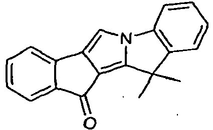 | 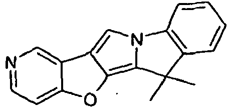 |
| 130 | 131 | 132 |
| 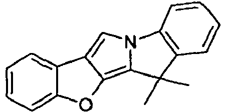 | 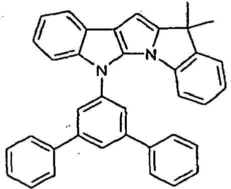 | 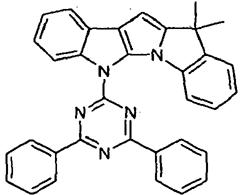 |
| 133 | 134 | 135 |
| 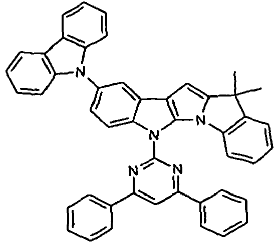 | 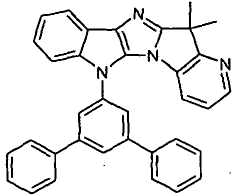 | 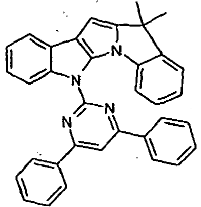 |
| 136 | 137 | 138 |
| 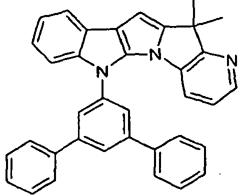 | 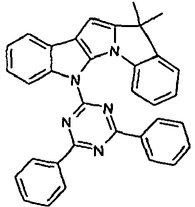 | 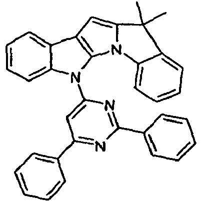 |
| 139 | 140 | 141 |
| 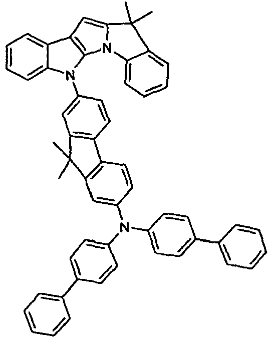 | 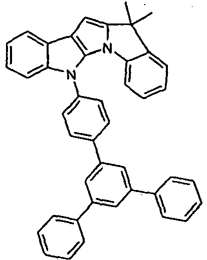 | 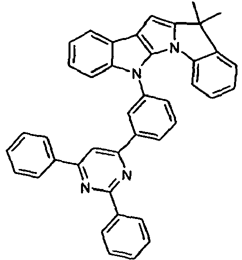 |
| 142 | 143 | 144 |
| 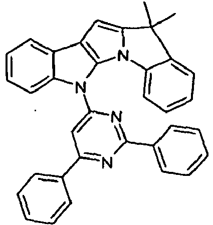 | 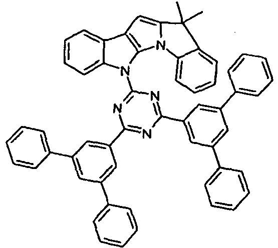 | 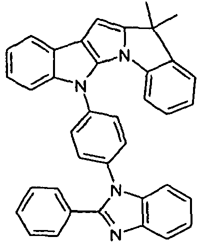 |
| 145 | 146 | 147 |
| 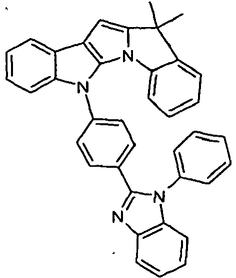 | 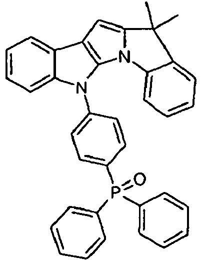 | 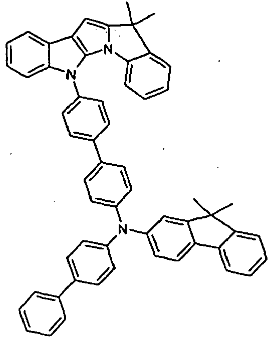 |
| 147 | 149 | 150 |
| 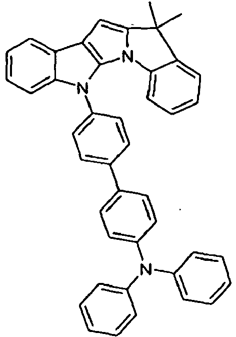 | 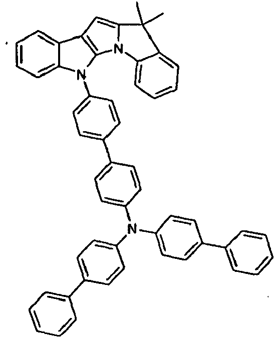 | 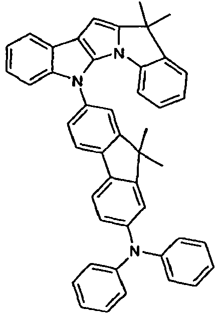 |
| 151 | 152 | 153 |
| 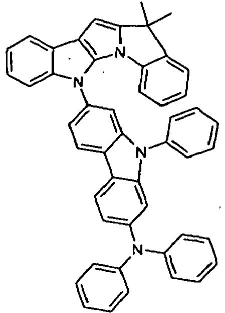 | 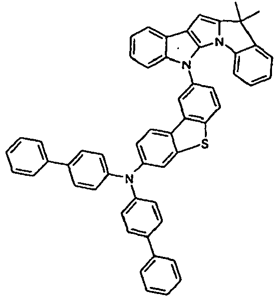 | 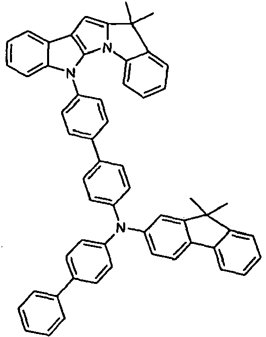 |
| 154 | 155 | 156 |
| 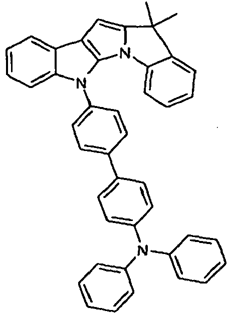 | 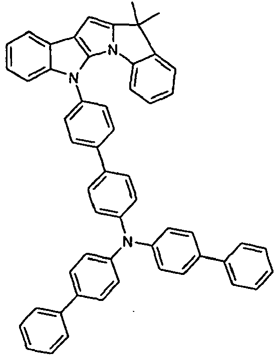 | 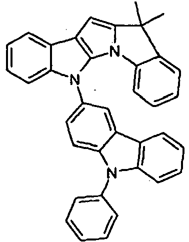 |
| 157 | 158 | 159 |
| 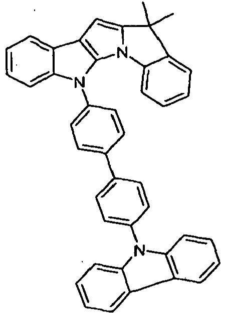 | 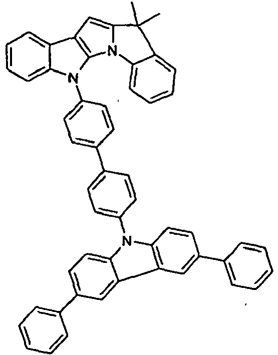 | 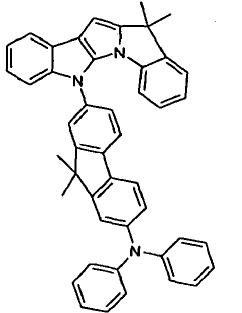 |
| 160 | 161 | 162 |
| 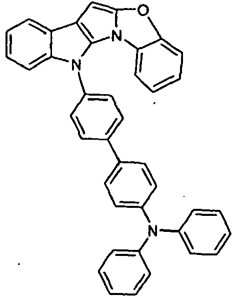 | 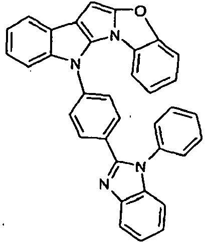 | 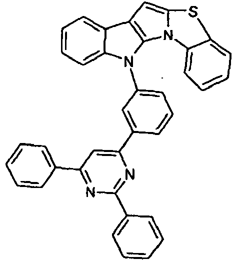 |
| 163 | 164 | 165 |
| 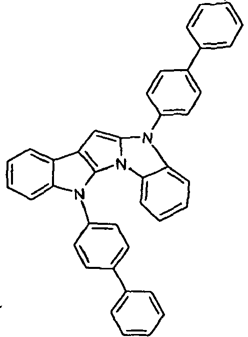 | 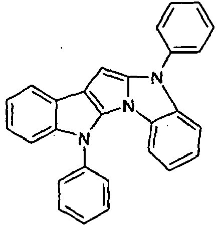 | 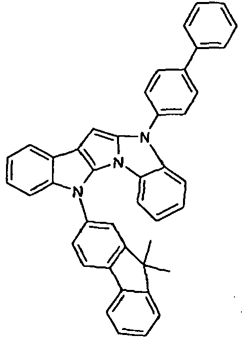 |
| 166 | 167 | 168 |

Die Synthese der erfindungsgemäßen Verbindungen kann durch dem Fachmann bekannte organische Syntheseverfahren, wie beispielsweise Hartwig-Buchwald-Reaktion und Friedel-Crafts-Alkylierung, erfolgen.

Im Folgenden werden als Beispiele verschiedene Synthesewege gezeigt, auf denen sich erfindungsgemäße Verbindungen herstellen lassen. Die erhaltenen Verbindungen können an beliebigen Positionen substituiert sein, gemäß der Definition der Verbindungen nach Formel (I), (II) bzw. (III). Der Übersichtlichkeit halber werden die Substituenten in den folgenden Schemata nicht explizit abgebildet, es sind also nur Grundkörper gezeigt. Dem Fachmann auf dem Gebiet der organischen Synthese ist bekannt, auf welche Weise, gegebenenfalls mit Hilfe von Schutzgruppentechnik, er die gewünschten Substituenten einführen kann.

In Schema 1 ist ein Verfahren zur Synthese einer erfindungsgemäßen Verbindung nach Formel (I) gezeigt, welche als divalente Gruppe X eine Gruppe NAr aufweist, und welche als divalente Gruppe Y eine Gruppe CR₂ aufweist. Dazu wird von der gezeigten kommerziell erhältlichen Pyrrolo-Indolverbindung ausgegangen, welche mit einem Phenylboronsäurederivat umgesetzt wird, so dass eine Kupplung zwischen dem Pyrrol-Stickstoffatom und der Phenylgruppe stattfindet. Der in vicinaler Position zum Pyrrol-Stickstoffatom vorliegende Carbonsäureester-Rest wird nun durch Addition einer lithiumorganischen Verbindung zum tertiären Alkohol reduziert, und es wird unter sauren Bedingungen eine Ringschlussreaktion mit der Phenylgruppe durchgeführt. In einer Hartwig-Buchwald-Kupplung kann nun eine Aryl- oder Heteroarylgruppe am Stickstoffatom des Indols eingeführt werden. 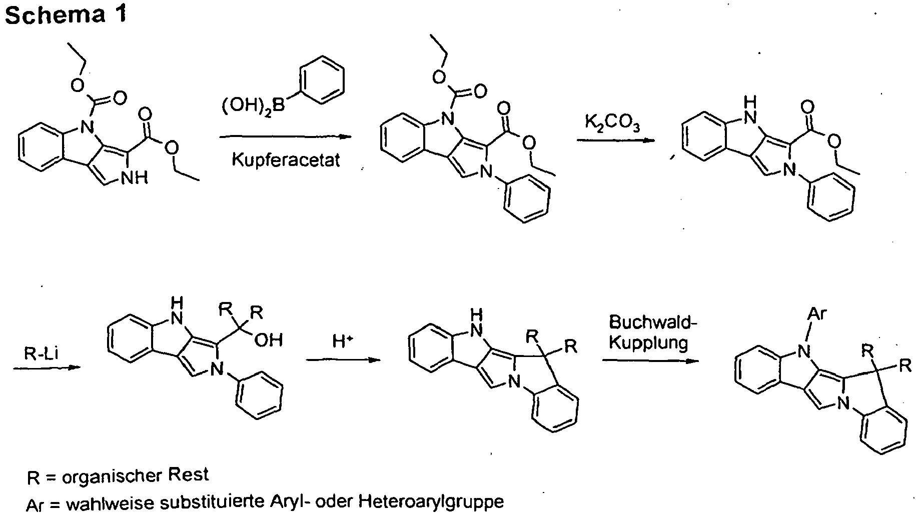 In Schema 2 ist ein Verfahren zur Synthese einer erfindungsgemäßen Verbindung nach Formel (I) gezeigt, welche als divalente Gruppe X eine Gruppe NAr aufweist, und welche als divalente Gruppe Y eine Gruppe O oder S aufweist. Das Verfahren unterscheidet sich von dem in Schema 1 Gezeigten im Wesentlichen dadurch, dass eine andere Ringschlussreaktion zur Einführung der Gruppe Y eingesetzt wird. Als Edukt wird ein Pyrrolo-Indolderivat eingesetzt, welches ein Fluoratom in vicinaler Position zum Pyrrol-Stickstoffatom aufweist. Es wird eine Phenylgruppe an das Pyrrol-Stickstoffatom gekuppelt, welche eine Gruppe -OH oder -SH in entsprechender Position aufweist. Die Ringschlussreaktion erfolgt nun zwischen der Gruppe -OH bzw. -SH und dem mit Fluor substituierten Kohlenstoffatom des Pyrrolrings unter stark basischen Bedingungen (NaH). Dabei wird die gewünschte O- bzw. S-Verbrückung hergestellt.

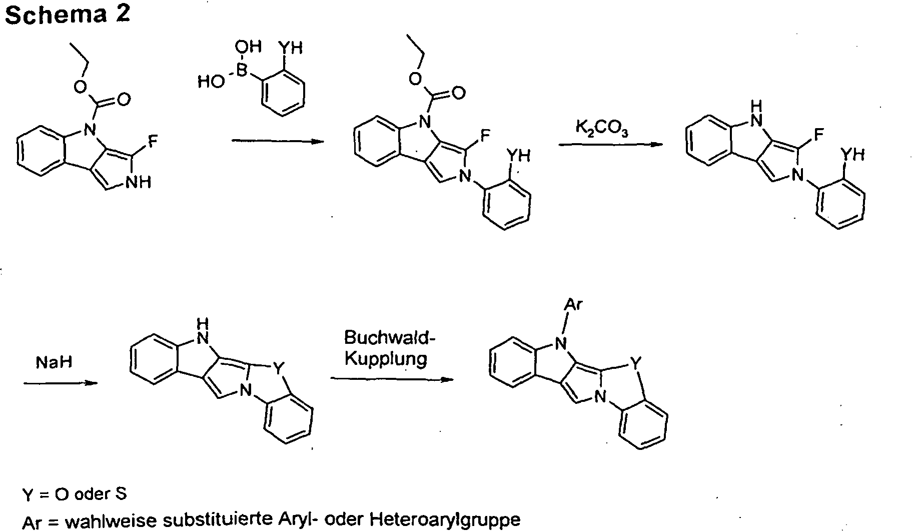

In Schema 3 ist ein Verfahren zur Synthese einer erfindungsgemäßen Verbindung nach Formel (II) gezeigt. Der Syntheseweg ist weitgehend analog zu dem in Schema 1 Gezeigten, mit dem Unterschied, dass als Edukt ein isomeres Pyrrolo-Indolderivat eingesetzt wird.

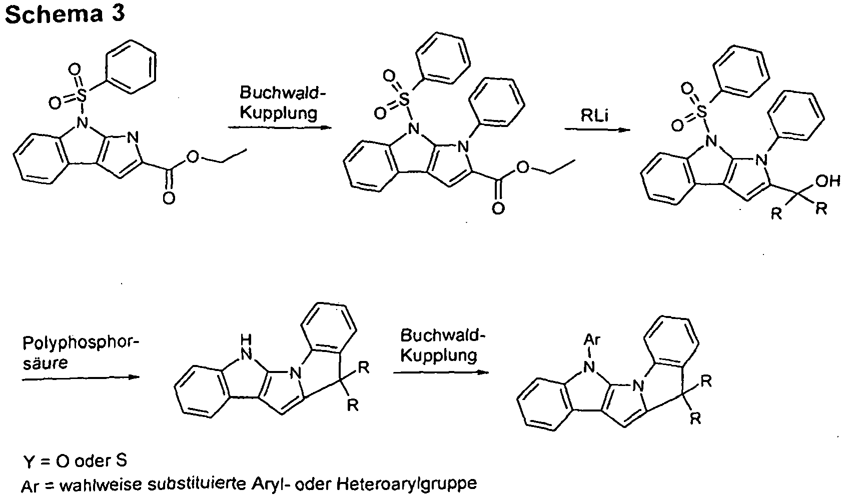

Die oben beschriebenen Synthesewege sollen lediglich als Beispiele dienen. Der Fachmann kann zur Synthese der erfindungsgemäßen Verbindungen auf alternative Syntheseverfahren zurückgreifen, wenn ihm dies unter den gegebenen Umständen vorteilhaft erscheint. Weiterhin kann er unter Heranziehung seines allgemeinen Fachwissens auf dem Gebiet der organischen Synthesechemie die gezeigten Synthesen erweitern und/oder modifizieren, um erfindungsgemäße Verbindungen herzustellen.

Weiterer Gegenstand der Erfindung ist somit ein Verfahren zur Herstellung einer Verbindung gemäß Formel (I), (II) oder (III), umfassend mindestens die folgenden Schritte:
(a) eine Kupplungsreaktion zwischen dem Pyrrol-Stickstoffatom und einer Aryl- oder Heteroarylgruppe; und
(b) eine Ringschlussreaktion zwischen dem Pyrrolring und der in Schritt (a) gekuppelten Aryl- oder Heteroarylgruppe.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität.

Weiterer Gegenstand der Erfindung sind daher Oligomere, Polymere oder Dendrimere enthaltend eine oder mehrere Verbindungen gemäß Formel (I), (II) oder (III), wobei die Bindung(en) zum Polymer, Oligomer oder Dendrimer an beliebigen, in Formel (I), (II) oder (III) mit R¹ oder R² substituierten Positionen lokalisiert sein können. Je nach Verknüpfung der Verbindung gemäß Formel (I), (II) oder (III) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nicht-konjugiert sein. Die erfindungsgemäßen Oligomere oder Polymere können linear, verzweigt oder dendritisch sein. In den linear verknüpften Strukturen können die Einheiten gemäß Formel (I), (II) oder (III) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe mitein-ander verknüpft sein. In verzweigten und dendritischen Strukturen können beispielsweise drei oder mehrere Einheiten gemäß Formel (I), (II) oder (III) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein. Für die Wiederholeinheiten gemäß Formel (I), (II) oder (III) in Oligomeren, Dendrimeren und Polymeren gelten dieselben Bevorzugungen wie oben für Verbindungen gemäß Formel (I), (II) oder (III) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 00/22026), Spirobifluorenen (z. B. gemäß EP 707020, EP 894107 oder WO 06/061181), Paraphenylenen (z. B. gemäß WO 92/18552), Carbazolen (z. B. gemäß WO 04/070772 oder WO 04/113468), Thiophenen (z. B. gemäß EP 1028136), Dihydrophenanthrenen (z. B. gemäß WO 05/014689 oder WO 07/006383), cis- und trans-Indenofluorenen (z. B. gemäß WO 04/041901 oder WO 04/113412), Ketonen (z. B. gemäß WO 05/040302), Phenanthrenen (z. B. gemäß WO 05/104264 oder WO 07/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 07/068325) oder phosphoreszierende Metallkomplexe (z. B. gemäß WO 06/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I), (II) oder (III) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Gegenstand der vorliegenden Erfindung ist somit auch ein Verfahren zur Herstellung der erfindungsgemäßen Polymere, Oligomere und Dendrimere, das dadurch gekennzeichnet ist, dass sie durch Polymerisation gemäß SUZUKI; Polymerisation gemäß YAMAMOTO, Polymerisation gemäß STILLE oder Polymerisation gemäß HARTWIG-BUCHWALD hergestellt werden. Die erfindungsgemäßen Dendrimere können gemäß dem Fachmann bekannten Verfahren oder in Analogie dazu hergestellt werden. Geeignete Verfahren sind in der Literatur beschrieben, wie z. B. in Frechet, Jean M. J.; Hawker, Craig J., "Hyperbranched polyphenylene and hyperbranched polyesters: new soluble, three-dimensional, reactive polymers", Reactive & Functional Polymers (1995), 26(1-3), 127-36; Janssen, H. M.; Meijer, E. W., "The synthesis and characterization of dendritic molecules", Materials Science and Technology (1999), 20 (Synthesis of Polymers), 403-458; Tomalia, Donald A., "Dendrimer molecules", Scientific American (1995), 272(5), 62-6; WO 2002/067343 A1 und WO 2005/026144 A1.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Miniemulsionen sein.

Gegenstand der Erfindung ist daher weiterhin eine Formulierung, insbesondere eine Lösung, Dispersion oder Miniemulsion, enthaltend mindestens eine Verbindung gemäß Formel (I), (II) oder (III) oder mindestens ein Polymer, Oligomer oder Dendrimer enthaltend mindestens eine Einheit gemäß Formel (I), (II) oder (III) sowie mindestens ein Lösungsmittel, bevorzugt ein organisches Lösungsmittel. Wie solche Lösungen hergestellt werden können, ist dem Fachmann bekannt und beispielsweise in den Anmeldungen WO 2002/072714 und WO 2003/019694 und der darin zitierten Literatur beschrieben.

Die erfindungsgemäßen Verbindungen gemäß Formel (I), (II) oder (III) eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und/oder Schichten eingesetzt.

Beispielsweise sind erfindungsgemäße Verbindungen, welche elektronenarme Gruppen wie Sechsring-Heteroarylgruppen mit einem oder mehreren Stickstoffatomen oder Fünfring-Heteroarylgruppen mit zwei oder mehr Heteroatomen enthalten, besonders zur Verwendung als Matrixmaterial für phosphoreszierende Dotanden, als Elektronentransportmaterial oder als Lochblockiermaterial geeignet.

Weiterhin sind erfindungsgemäße Verbindungen, welche mit aromatischen oder heteroaromatischen Ringsystemen mit 5 bis 20 aromatischen Ringatomen, insbesondere mit aromatischen Ringsystemen mit 12 bis 20 aromatischen Ringatomen, und/oder mit einer oder mehreren Arylaminogruppen substituiert sind, besonders zur Verwendung als Lochtransportmaterialien oder zur Verwendung als fluoreszierende Dotanden geeignet.

Bevorzugt werden die erfindungsgemäßen Verbindungen als Matrixmaterial in einer emittierenden Schicht, als Lochtransportmaterial in einer Lochtransportschicht oder als Elektronentransportmaterial in einer Elektronentransportschicht eingesetzt. Weiterhin bevorzugt werden die erfindungsgemäßen Verbindungen in einer Lochinjektionsschicht eingesetzt. Sie können aber auch in anderen Schichten und/oder Funktionen eingesetzt werden, beispielsweise als fluoreszierende Dotanden in einer emittierenden Schicht oder als Loch- oder Elektronenblockiermaterialien.

Ein weiterer Gegenstand der Erfindung ist daher die Verwendung der erfindungsgemäßen Verbindungen in elektronischen Vorrichtungen. Dabei sind die elektronischen Vorrichtungen bevorzugt ausgewählt aus der Gruppe bestehend aus organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und besonders bevorzugt ausgewählt aus organischen Elektrolumineszenzvorrichtungen (OLEDs).

Nochmals ein weiterer Gegenstand der Erfindung sind elektronische Vorrichtungen, enthaltend mindestens eine Verbindung gemäß Formel (I), (II) oder (III). Dabei sind die elektronischen Vorrichtungen bevorzugt ausgewählt aus den oben genannten Vorrichtungen. Besonders bevorzugt sind organische Elektrolumineszenzvorrichtungen, enthaltend Anode, Kathode und mindestens eine emittierende Schicht, dadurch gekennzeichnet, dass mindestens eine organische Schicht, die eine emittierende Schicht, eine Elektronentransportschicht oder eine andere Schicht sein kann, mindestens eine Verbindung gemäß Formel (I), (II) oder (III) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Ladungserzeugungsschichten (Charge-Generation Layers) (IDMC 2003, Taiwan; Session 21 OLED (5), T. Matsumoto, T. Nakada, J. Endo,. K. Mori, N. Kawamura, A. Yokoi, J. Kido, Multiphoton Organic EL Device Having Charge Generation Layer)*,* Auskopplungsschichten und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt. Die in den jeweiligen Schichten und Funktionen bevorzugt eingesetzten Verbindungen werden in späteren Abschnitten explizit offenbart.

Es ist erfindungsgemäß bevorzugt, wenn die Verbindung gemäß Formel (I), (II) oder (III) in einer elektronische Vorrichtung enthaltend einen oder mehrere phosphoreszierende Dotanden eingesetzt wird. Dabei kann die Verbindung in unterschiedlichen Schichten, bevorzugt in einer Elektronentransportschicht, einer Lochtransportschicht, einer Loch-injektionsschicht oder in der emittierenden Schicht verwendet werden. Die Verbindung gemäß Formel (I), (II) oder (III) kann aber auch erfindungsgemäß in einer elektronischen Vorrichtung, enthaltend einen oder mehrere fluoreszierende Dotanden und keine phosphoreszierenden Dotanden, eingesetzt werden.

Als phosphoreszierende Dotanden (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele der oben beschriebenen phosphoreszierenden Dotanden können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den erfindungsgemäßen Verbindungen in organischen Elektrolumineszenzvorrichtungen einsetzen.

Weitere Beispiele für geeignete phosphoreszierende Dotanden können der in einem späteren Abschnitt folgenden Tabelle entnommen werden.

In einer bevorzugten Ausführungsform der vorliegenden Erfindung werden die Verbindungen der Formel (I), (II) oder (III) als Matrixmaterial in Kombination mit einem oder mehreren Dotanden, vorzugsweise phosphoreszierenden Dotanden, eingesetzt. Die Verbindungen sind insbesondere dann zur Verwendung als Matrixmaterial geeignet, wenn sie eine oder mehrere elektronenarme Gruppen enthalten, wie zum Beispiel Sechsring-Heteroarylgruppen mit einem oder mehreren Stickstoffatomen oder Fünfring-Heteroarylgruppen mit zwei oder mehr Stickstoffatomen.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%. Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindungen gemäß Formel (I), (II) oder (III) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:6 bis 1:1 vorliegen. Bevorzugt werden Mixed-Matrix-Systeme in phosphoreszierenden organischen Elektrolumineszenzvorrichtungen eingesetzt.

Die Mixed-Matrix-Systeme können einen oder mehrere Dotanden umfassen. Die Dotandverbindung bzw. die Dotandverbindungen zusammen haben erfindungsgemäß einen Anteil von 0.1 bis 50.0 Vol.-% an der Gesamtmischung und bevorzugt einen Anteil von 0.5 bis 20.0 Vol.-% an der Gesamtmischung. Entsprechend haben die Matrixkomponenten zusammen einen Anteil von 50.0 bis 99.9 Vol-% an der Gesamtmischung und bevorzugt einen Anteil von 80.0 bis 99.5 Vol.-% an der Gesamtmischung.

Besonders geeignete Matrixmaterialien, welche in Kombination mit den erfindungsgemäßen Verbindungen als Matrixkomponenten eines Mixed-Matrix-Systems eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, Indenocarbazolderivate, z. B. gemäß WO 2010/136109 und WO 2011/000455, oder verbrückte Carbazole, *z*. B gemäß WO 2011/088877 und WO 2011/128017.

Bevorzugte phosphoreszierende Dotanden zur Verwendung in Mixed-Matrix-Systemen enthaltend die erfindungsgemäßen Verbindungen sind die in einer folgenden Tabelle aufgeführten phosphoreszierenden Dotanden.

In einer weiteren bevorzugten Ausführungsform der Erfindung werden die Verbindungen gemäß Formel (I), (II) oder (III) als Lochtransportmaterial eingesetzt. Die Verbindungen werden dann bevorzugt in einer Lochtransportschicht und/oder in einer Lochinjektionsschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen der Lochinjektionsschicht und der Emissionsschicht liegt. Die Verbindungen sind insbesondere dann zur Verwendung als Lochtransportmaterial geeignet, wenn sie mit einem oder mehreren aromatischen Ringsystemen mit 12 bis 20 aromatischen Ringatomen und/oder mit einer oder mehreren Arylaminogruppen substituiert sind.

Wird die Verbindung gemäß Formel (I), (II) oder (III) als Lochtransportmaterial in einer Lochtransportschicht eingesetzt, so kann die Verbindung als Reinmaterial, d.h. in einem Anteil von 100 % in der Lochtransportschicht eingesetzt werden, oder sie kann in Kombination mit weiteren Verbindungen, insbesondere p-Dotanden, in der Lochtransportschicht eingesetzt werden.

In einer weiteren Ausführungsform der Erfindung werden die die Verbindungen gemäß Formel (I), (II) oder (III) als fluoreszierende Dotanden in einer emittierenden Schicht eingesetzt. Die Verbindungen sind insbesondere dann zur Verwendung als fluoreszierende Dotanden geeignet, wenn sie mit einem oder mehreren aromatischen Systemen, bevorzugt aromatischen Systemen enthaltend 12 bis 20 aromatische Ringatome, substituiert sind. Die erfindungsgemäßen Verbindungen werden bevorzugt als grüne oder blaue Emitter verwendet.

Der Anteil der Verbindung gemäß Formel (I), (II) oder (III) als Dotand in der Mischung der emittierenden Schicht beträgt in diesem Fall zwischen 0.1 und 50.0 Vol.-%, bevorzugt zwischen 0.5 und 20.0 Vol.-%, besonders bevorzugt zwischen 0.5 und 8.0 Vol.-%. Entsprechend beträgt der Anteil des Matrixmaterials zwischen 50.0 und 99.9 Vol.-%, bevorzugt zwischen 80.0 und 99.5 Vol.-%, besonders bevorzugt zwischen 92.0 und 99.5 Vol.-%.

Bevorzugte Matrixmaterialien zur Verwendung in Kombination mit den erfindungsgemäßen Verbindungen als fluoreszierende Dotanden sind in einem der folgenden Abschnitte aufgeführt. Sie entsprechen den als bevorzugt aufgeführten Matrixmaterialien für fluoreszierende Dotanden.

In einer weiteren Ausführungsform der Erfindung werden die Verbindungen als Elektronentransportmaterialien in einer Elektronentransportschicht einer organischen Elektrolumineszenzvorrichtung eingesetzt. In diesem Fall ist es bevorzugt, dass die erfindungsgemäßen Verbindungen eine oder mehrere elektronenarme Gruppen wie z. B. Sechsring-Heteroarylgruppen mit einem oder mehreren Stickstoffatomen oder Fünfring-Heteroarylgruppen mit zwei oder mehr Heteroatomen aufweisen.

Die organische Elektrolumineszenzvorrichtung kann auch mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren, wobei die verschiedenen Farben in dieser Ausführungsform der Erfindung zusammen weißes Licht ergeben. Insbesondere bevorzugt sind Dreischichtsysteme, also Systeme mit drei emittierenden Schichten, wobei eine oder mehrere dieser Schichten eine Verbindung gemäß Formel (I), (II) oder (III) enthalten kann und wobei die drei Schichten blaue, grüne und orangefarbene oder rote Emission zeigen (für den prinzipiellen Aufbau siehe z. B. WO 2005/011013). Ebenso eignen sich in solchen Systemen für weiße Emission Emitter, welche breitbandige Emissionsbanden aufweisen und dadurch weiße Emission zeigen. Alternativ und/oder zusätzlich können die erfindungsgemäßen Verbindungen in solchen Systemen auch in einer Lochtransportschicht oder Elektronentransportschicht oder in einer anderen Schicht vorhanden sein.

Im Folgenden werden die in den elektronischen Vorrichtungen enthaltend eine oder mehrere erfindungsgemäße Verbindungen bevorzugt eingesetzten weiteren Funktionsmaterialien aufgeführt.

Besonders geeignete phosphoreszierende Dotanden stellen die in der folgenden Tabelle aufgeführten Verbindungen dar.

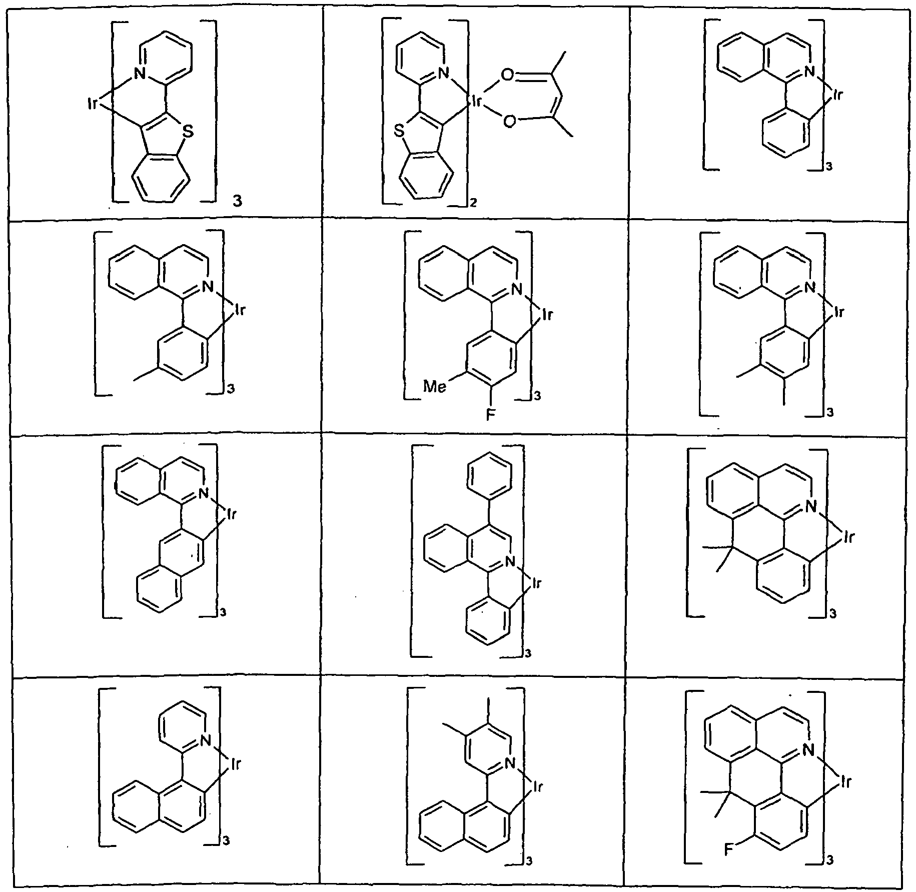

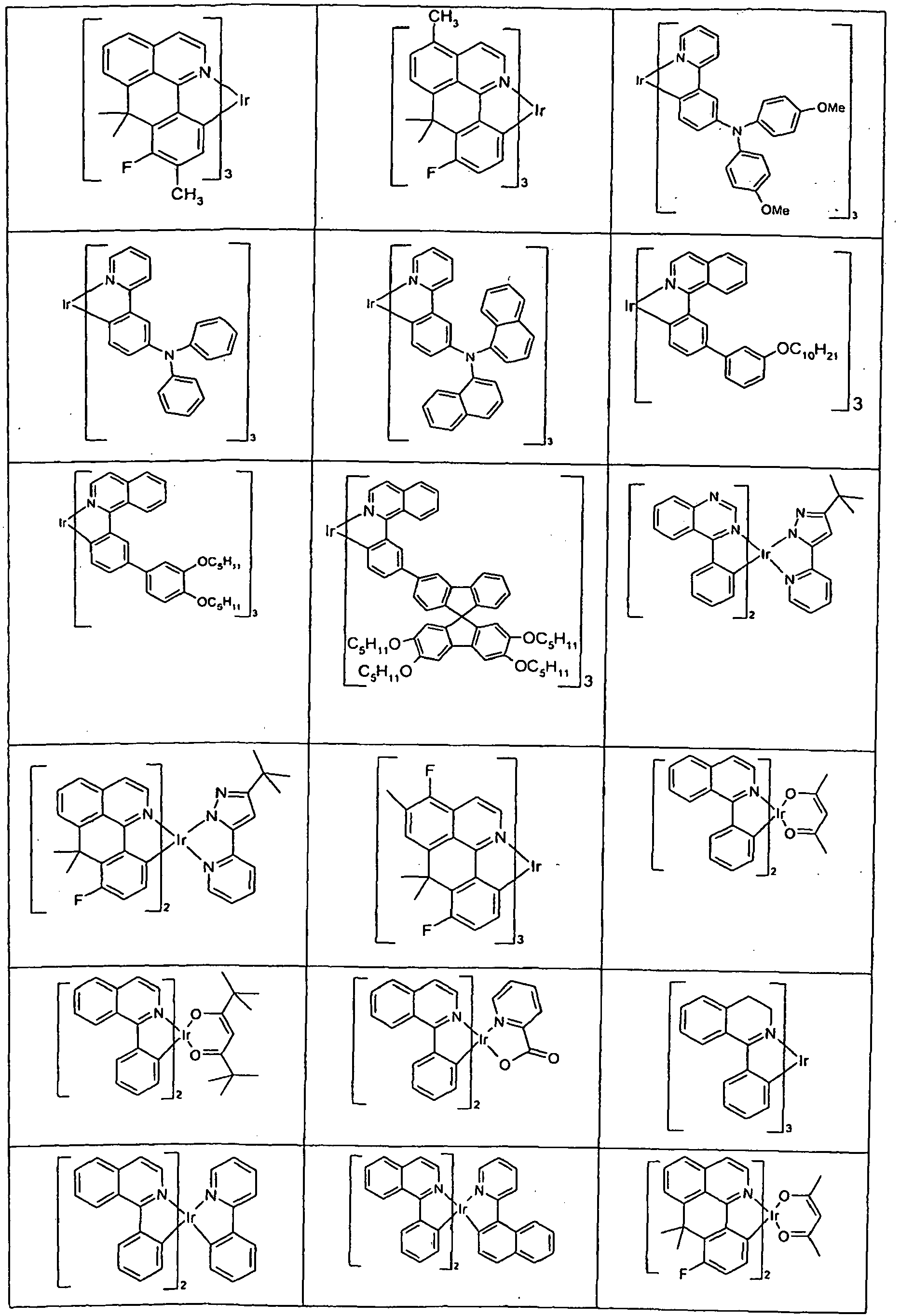

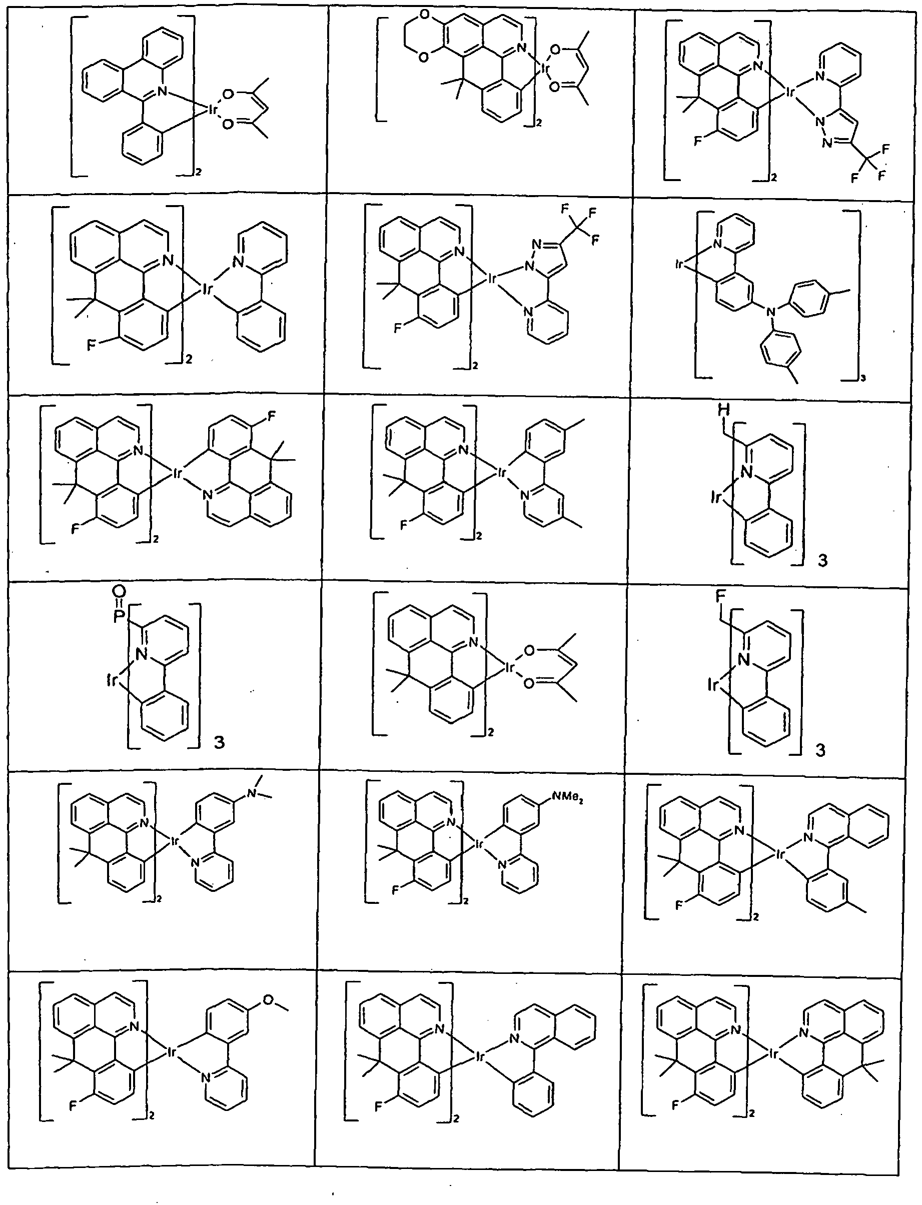

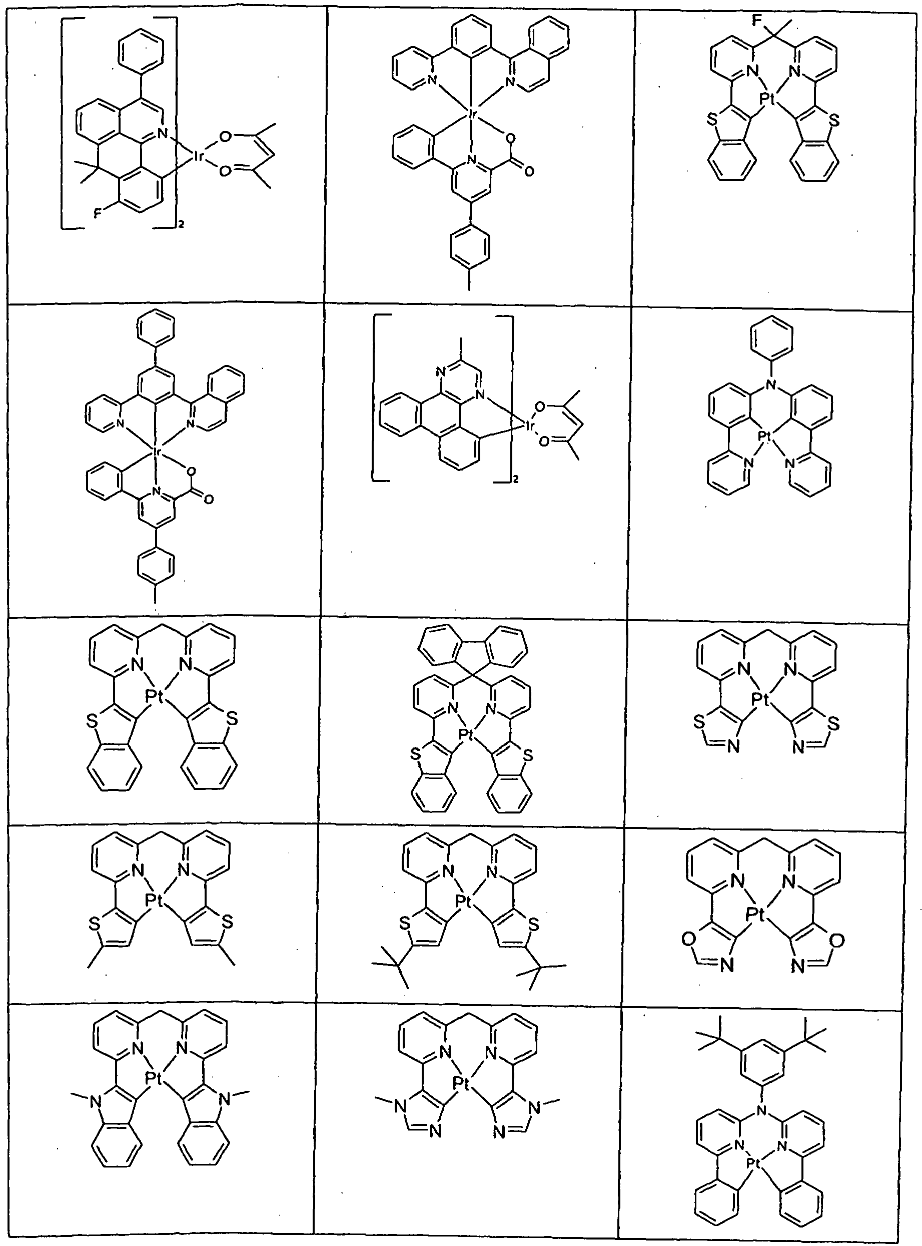

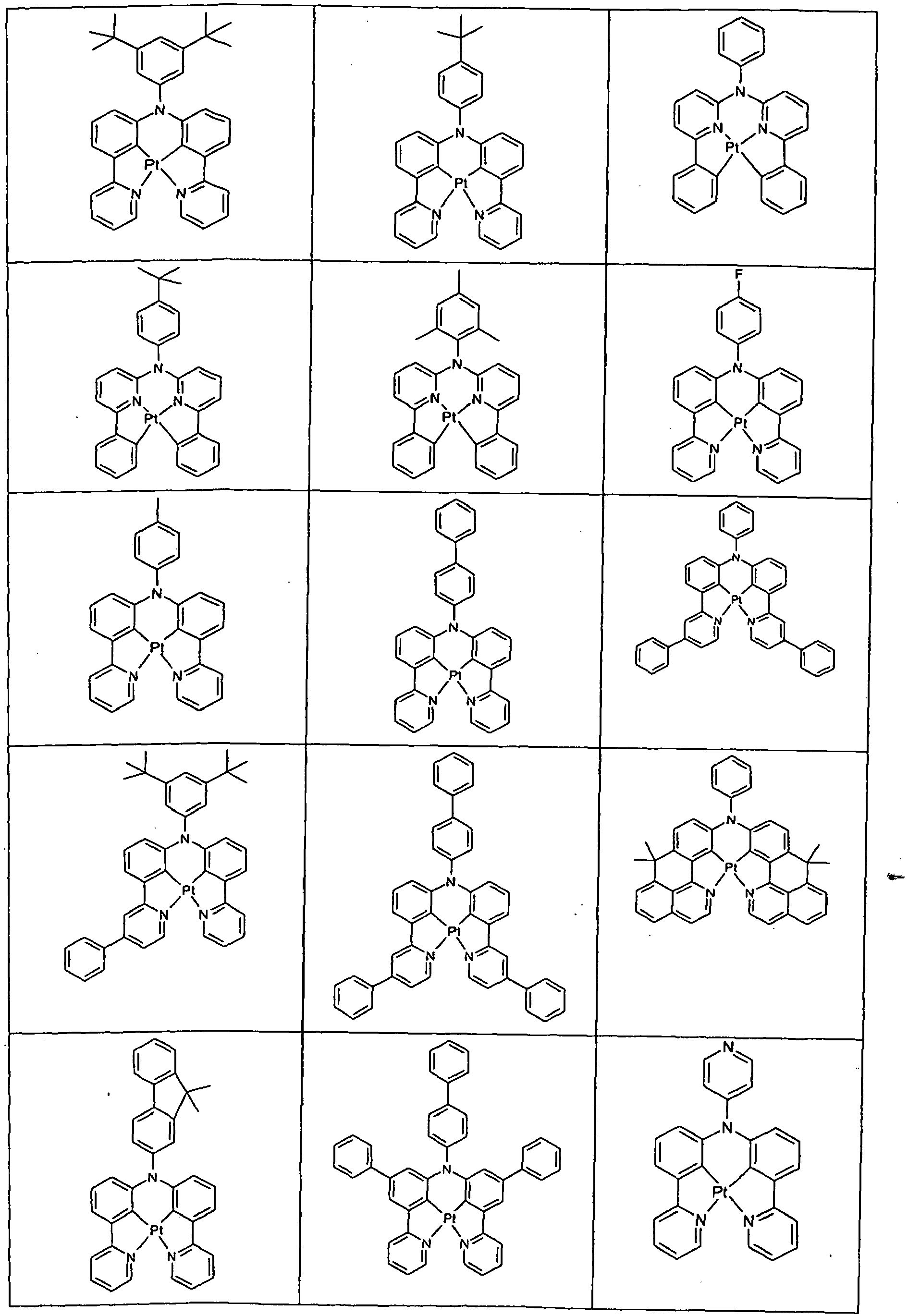

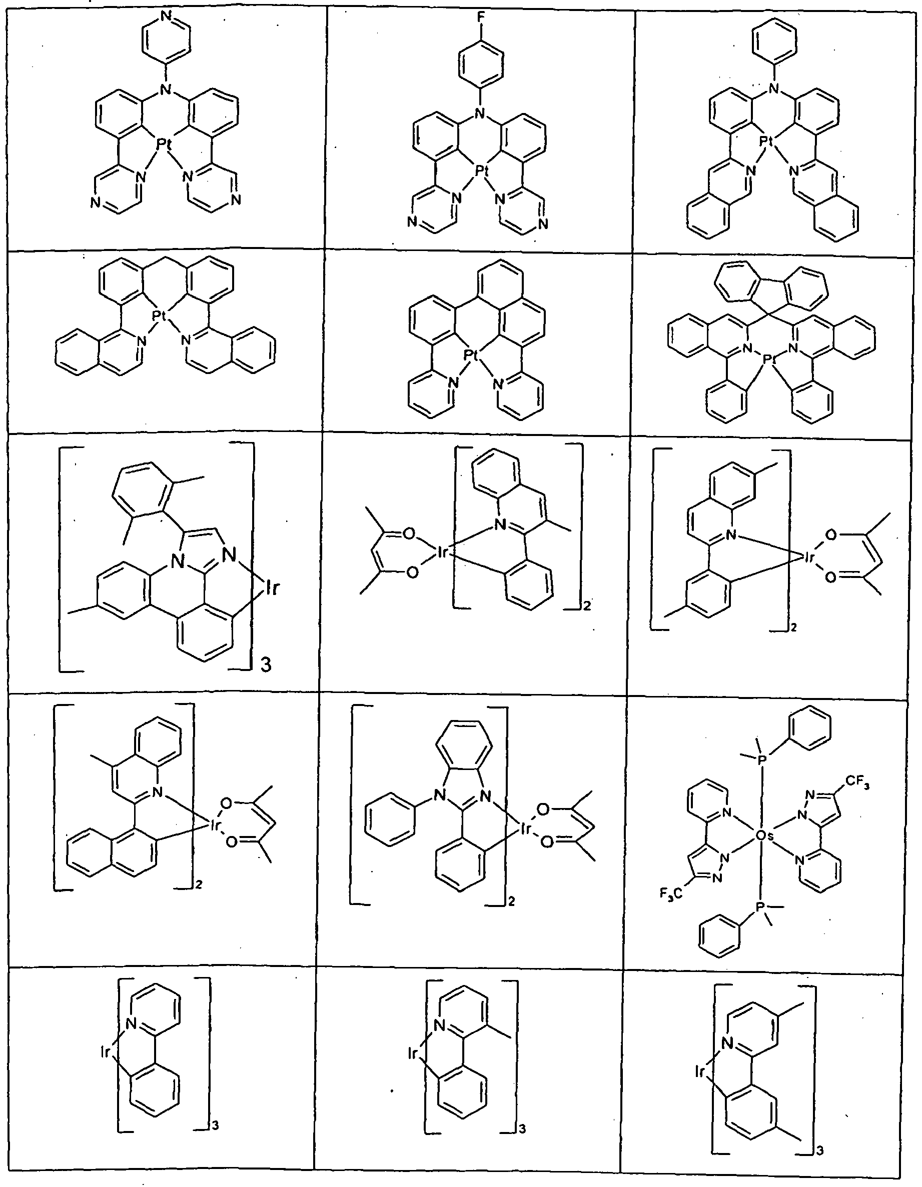

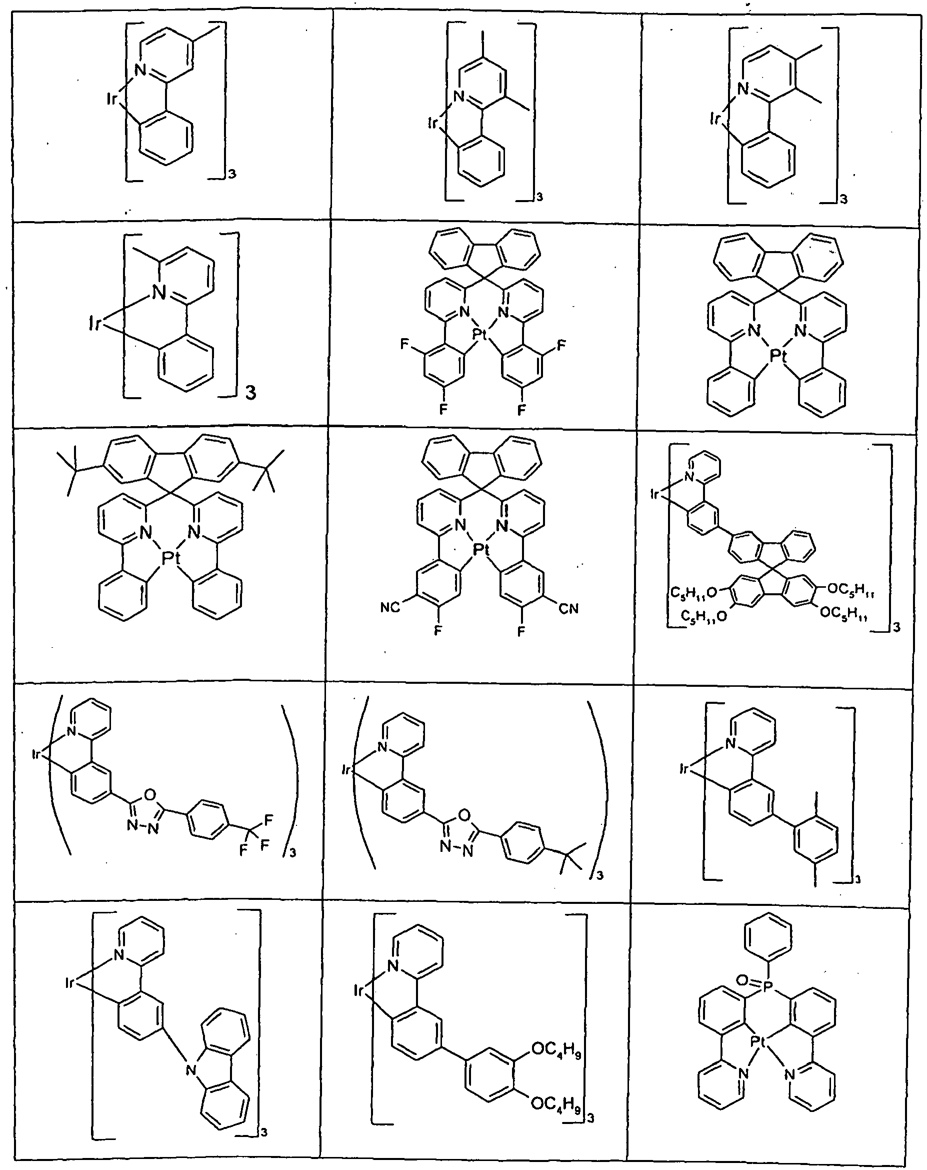

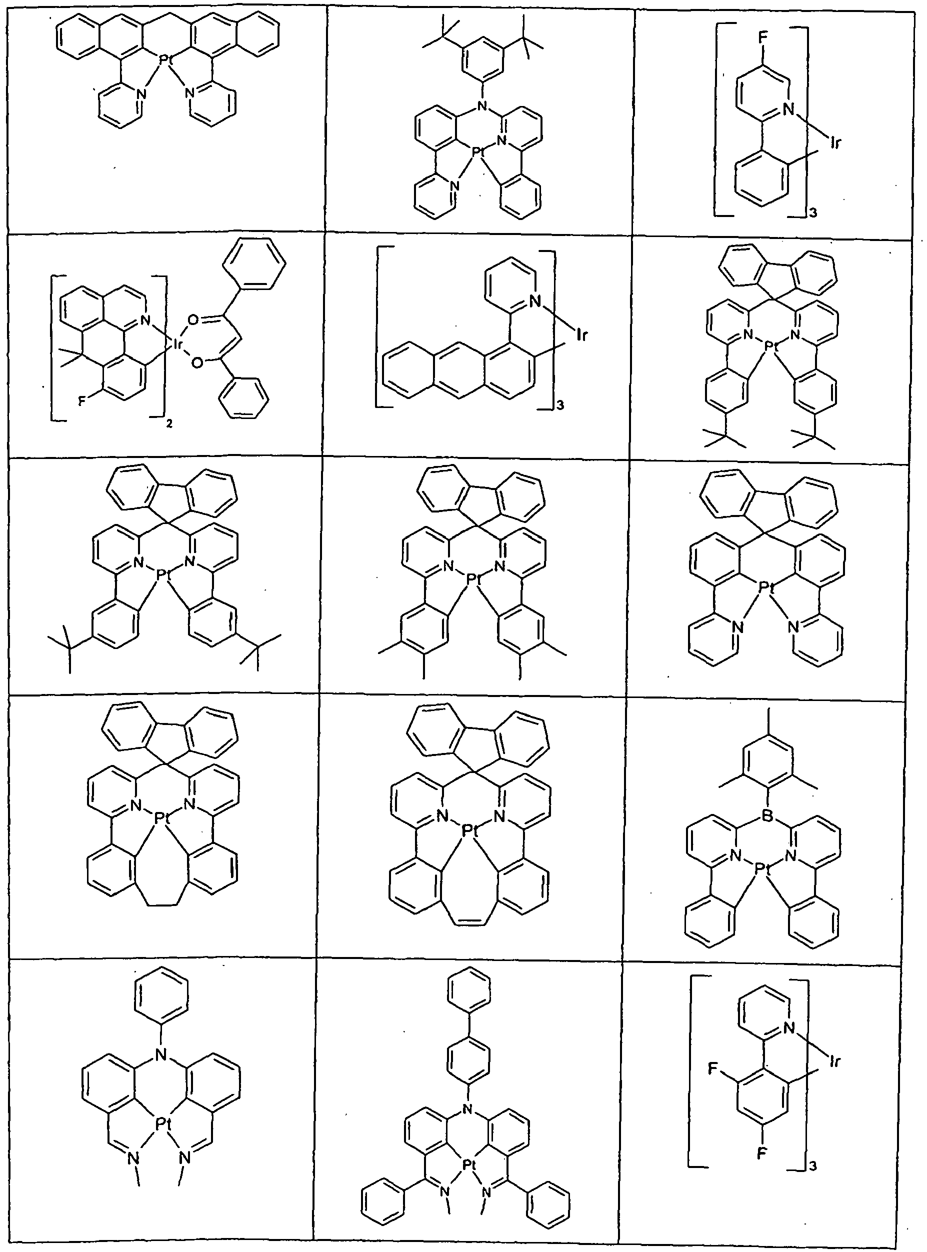

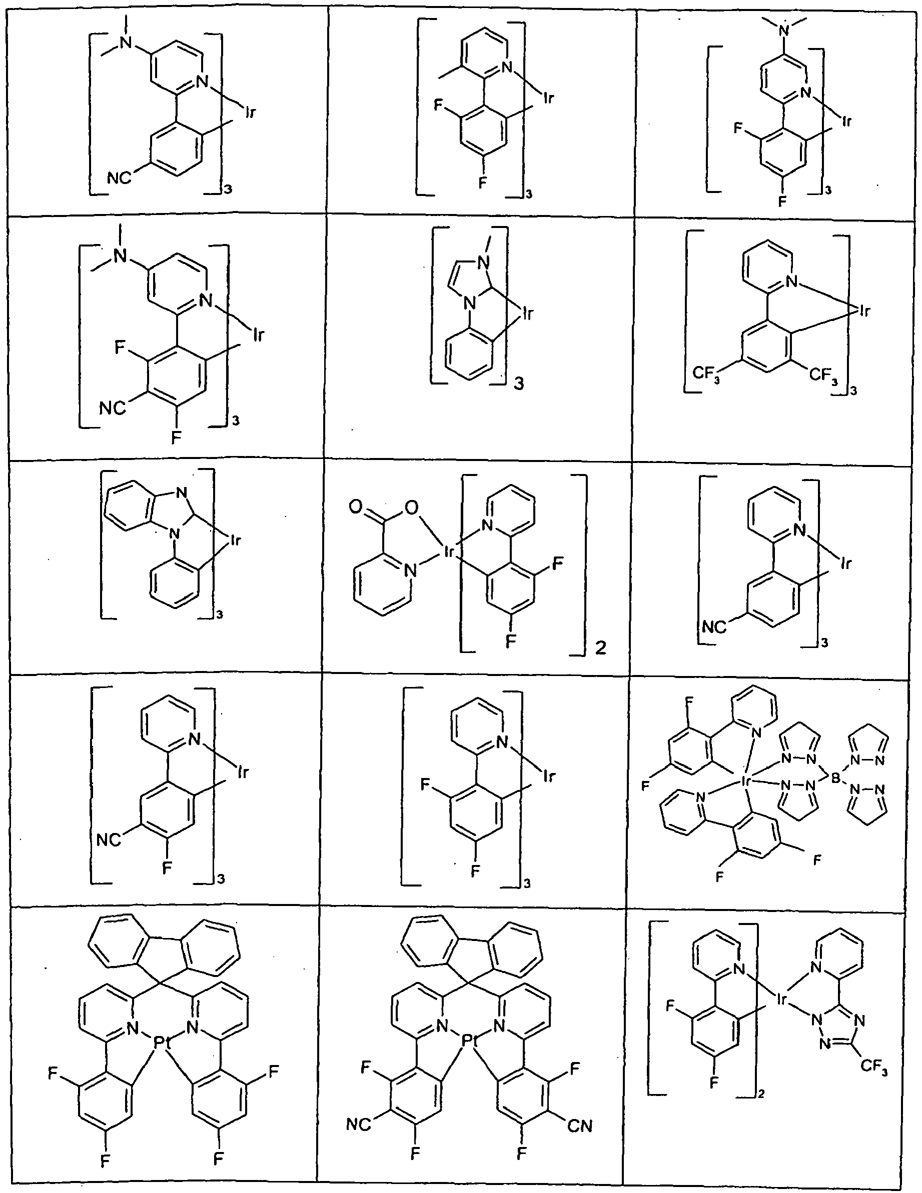

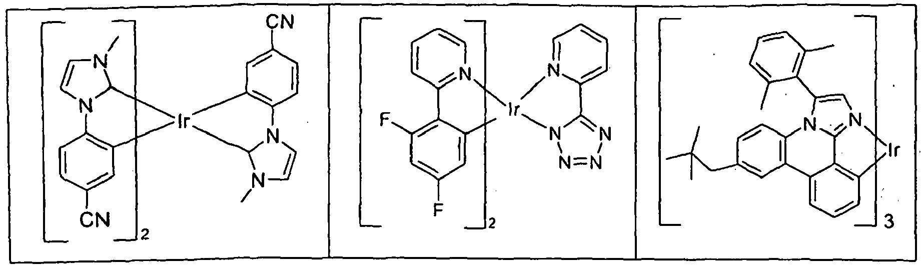

Bevorzugte fluoreszierende Dotanden sind ausgewählt aus der Klasse der Monostyrylamine, der Distyrylamine, der Tristyrylamine, der Tetrastyrylamine, der Styrylphosphine, der Styrylether und der Arylamine. Unter einem Monostyrylamin wird eine Verbindung verstanden, die eine substituierte oder unsubstituierte Styrylgruppe und mindestens ein, bevorzugt aromatisches, Amin enthält. Unter einem Distyrylamin wird eine Verbindung verstanden, die zwei substituierte oder unsubstituierte Styrylgruppen und mindestens ein, bevorzugt aromatisches, Amin enthält. Unter einem Tristyrylamin wird eine Verbindung verstanden, die drei substituierte oder unsubstituierte Styrylgruppen und mindestens ein, bevorzugt aromatisches, Amin enthält. Unter einem Tetrastyrylamin wird eine Verbindung verstanden, die vier substituierte oder unsubstituierte Styrylgruppen und mindestens ein, bevorzugt aromatisches, Amin enthält. Die Styrylgruppen sind besonders bevorzugt Stilbene, die auch noch weiter substituiert sein können. Entsprechende Styrylphosphine und Styrylether sind in Analogie zu den Aminen definiert. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte fluoreszierende Dotanden sind gewählt aus Indenofluorenaminen bzw. -diaminen, beispielsweise gemäß WO 2006/122630, Benzoindenofluorenaminen bzw. -diaminen, beispielsweise gemäß WO 2008/006449, und Dibenzoindenoflüorenaminen bzw. -diaminen, beispielsweise gemäß WO 2007/140847. Beispiele für fluoreszierende Dotanden aus der Klasse der Styrylamine sind substituierte oder unsubstituierte Tristilbenamine oder die fluoreszierenden Dotanden, die in WO 2006/000388, WO 2006/058737, WO 2006/000389, WO 2007/065549. und WO 2007/115610 beschrieben sind. Weiterhin bevorzugt sind die in DE 102008035413 offenbarten kondensierten Kohlenwasserstoffe. Weiterhin können die Verbindungen gemäß Formel (I), (II) oder (III) als fluoreszierende Dotanden verwendet werden.

Geeignete fluoreszierende Dotanden sind weiterhin die in der folgenden Tabelle abgebildeten Strukturen, sowie die in JP 2006/001973, WO 2004/047499, WO 2006/098080, WO 2007/065678, US 2005/0260442 und WO 2004/092111 offenbarten Derivate dieser Strukturen.

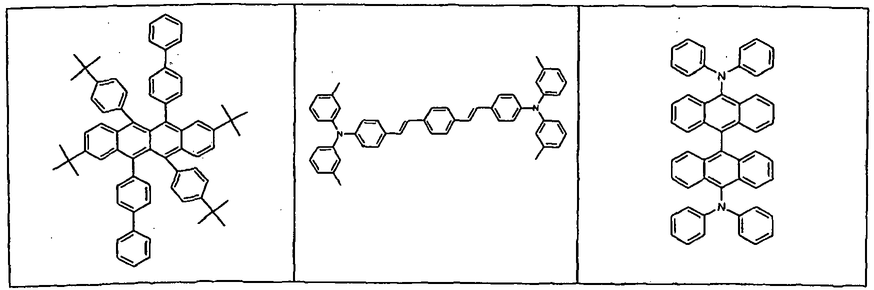

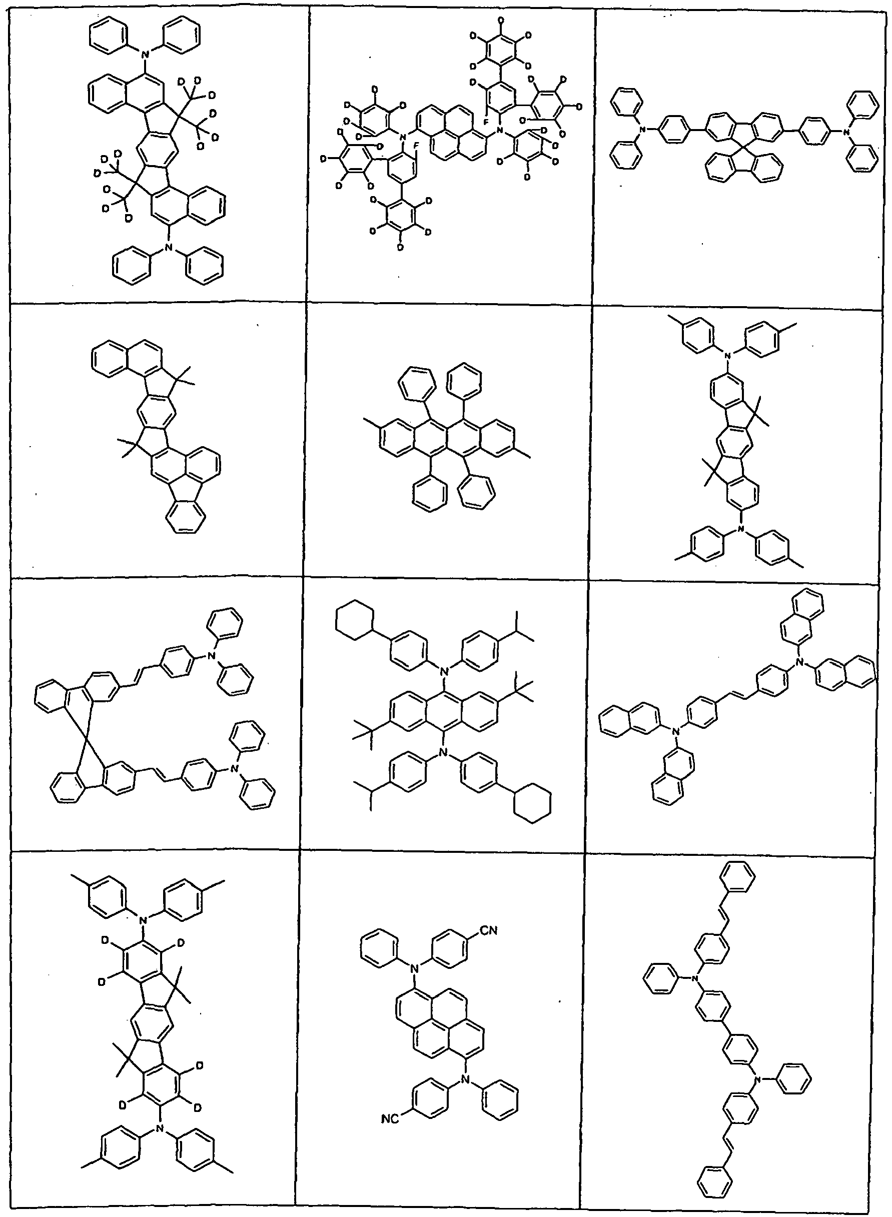

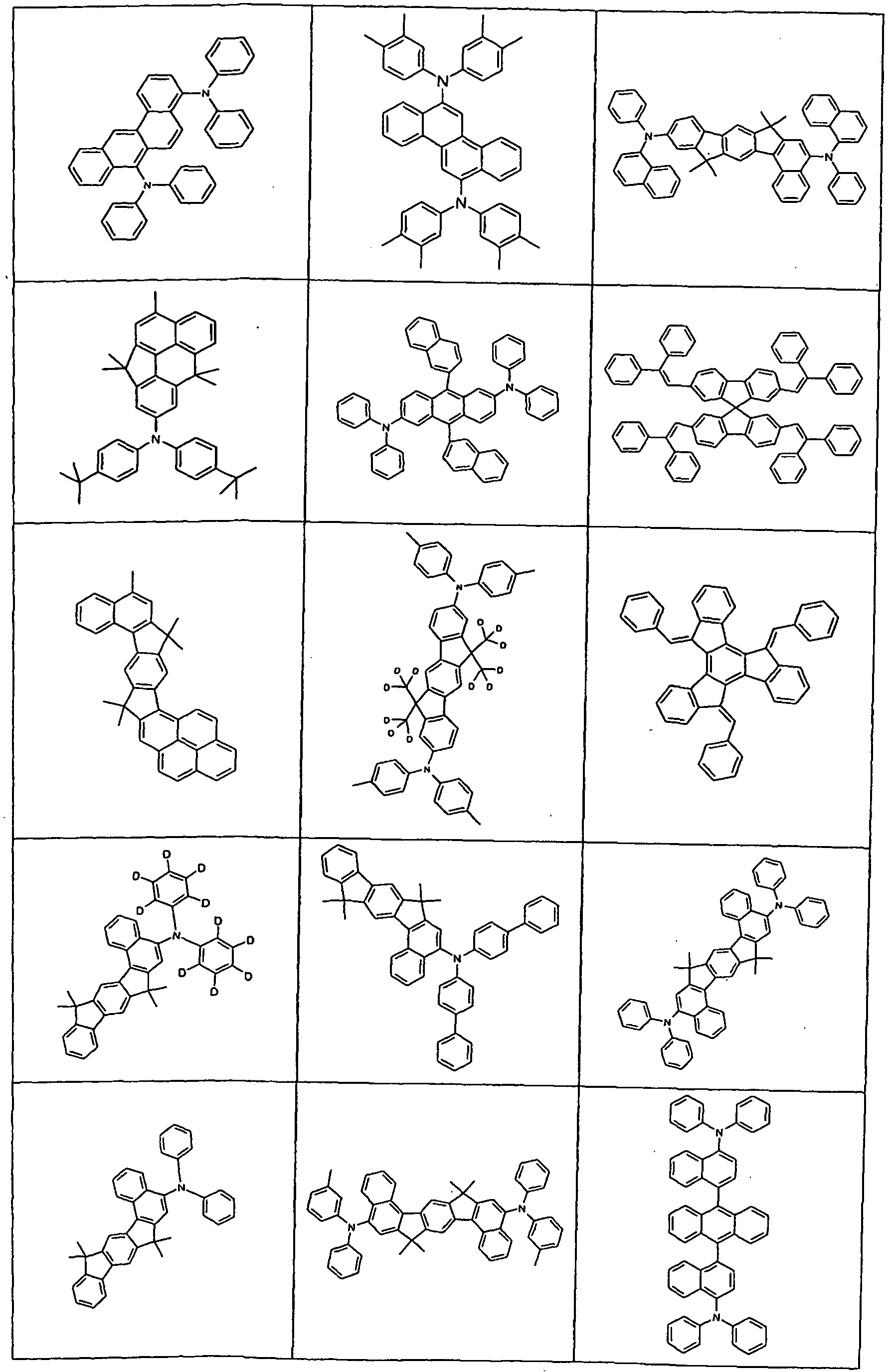

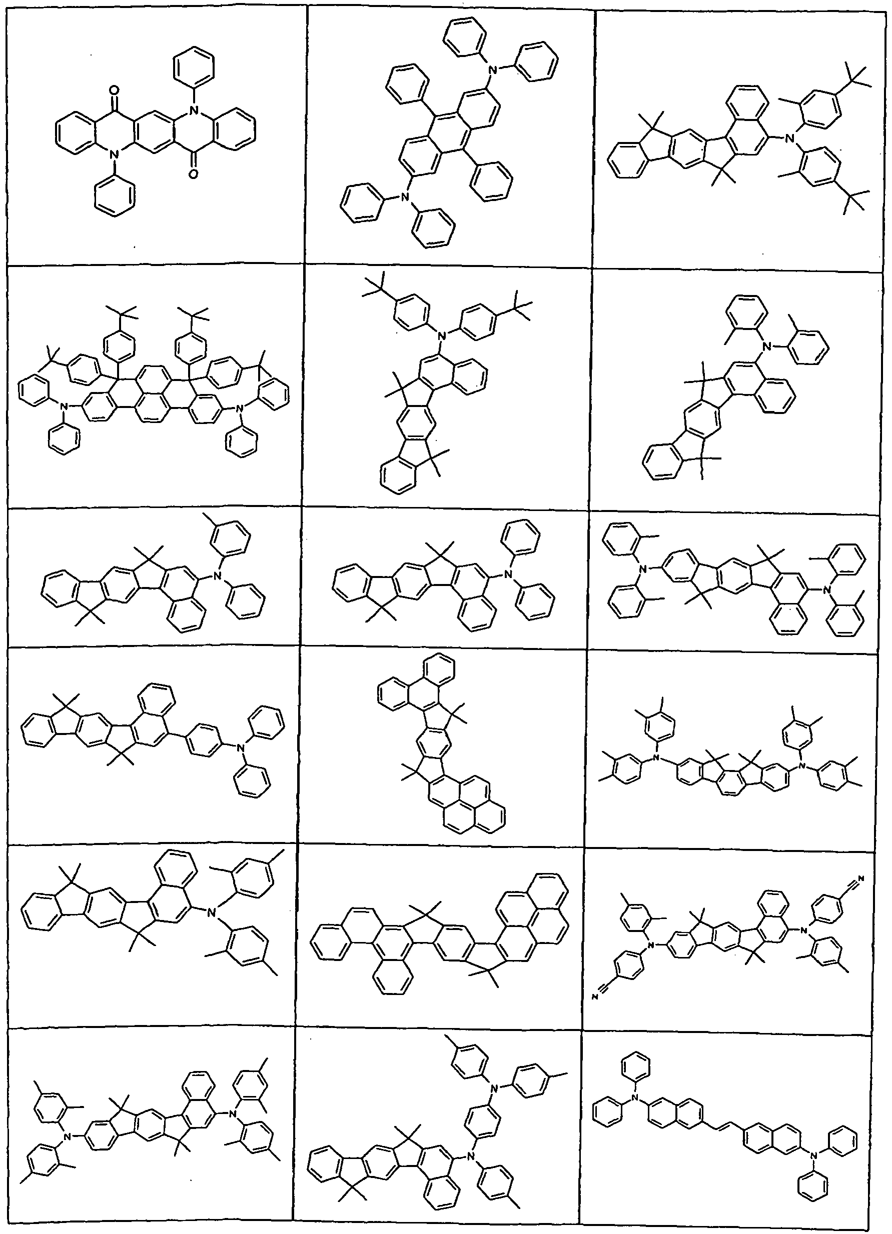

Als Matrixmaterialien, bevorzugt für fluoreszierende Dotanden, kommen Materialien verschiedener Stoffklassen in Frage. Bevorzugte Matrixmaterialien sind ausgewählt aus den Klassen der Oligoarylene (z. B. 2,2',7,7'-Tetrapheny(spirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß. WO 2004/081017), der lochleitenden Verbindungen (z: B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Weiterhin kommen als Matrixmaterialien bevorzugt die erfindungsgemäßen Verbindungen in Frage. Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrix-materialien sind ausgewählt aus den Klassen der Oligoarylene, enthaltend Anthracen, Benzanthracen, Benzphenanthren und/oder Pyren oder Atropisomere dieser Verbindungen. Unter einem Oligoaryfen im Sinne dieser Erfindung soll eine Verbindung verstanden werden, in der mindestens drei Aryl- bzw. Arylengruppen aneinander gebunden sind.

Geeignete Matrixmaterialien, bevorzugt für fluoreszierende Dotanden, sind beispielsweise die in der folgenden Tabelle abgebildeten Materialien, sowie Derivate dieser Materialien, wie sie in WO 2004/018587, WO 2008/006449, US 5935721, US 2005/0181232, JP 2000/273056, EP 681019, US 2004/0247937 und US 2005/0211958 offenbart werden.

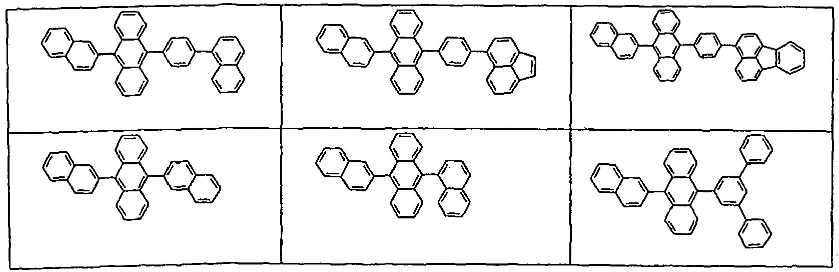

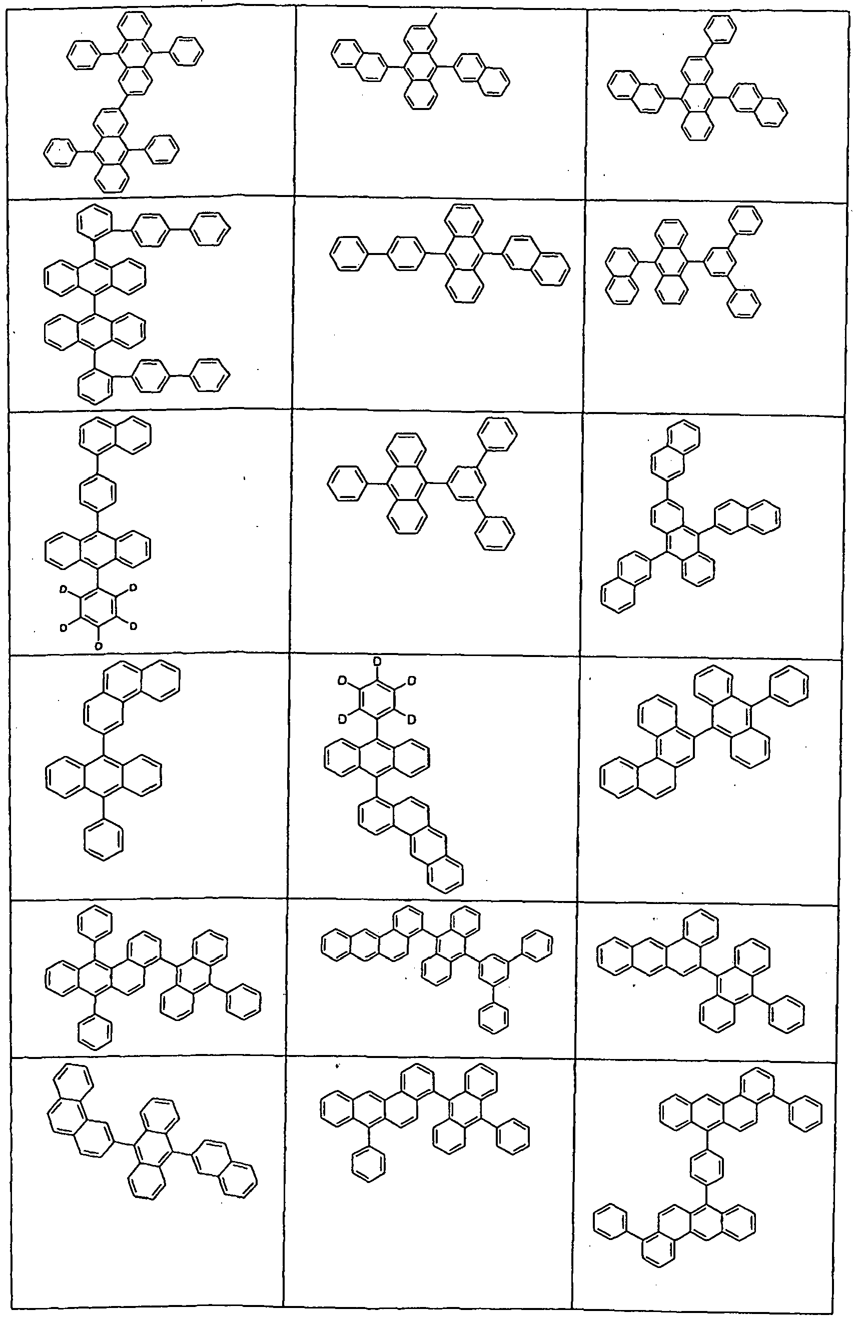

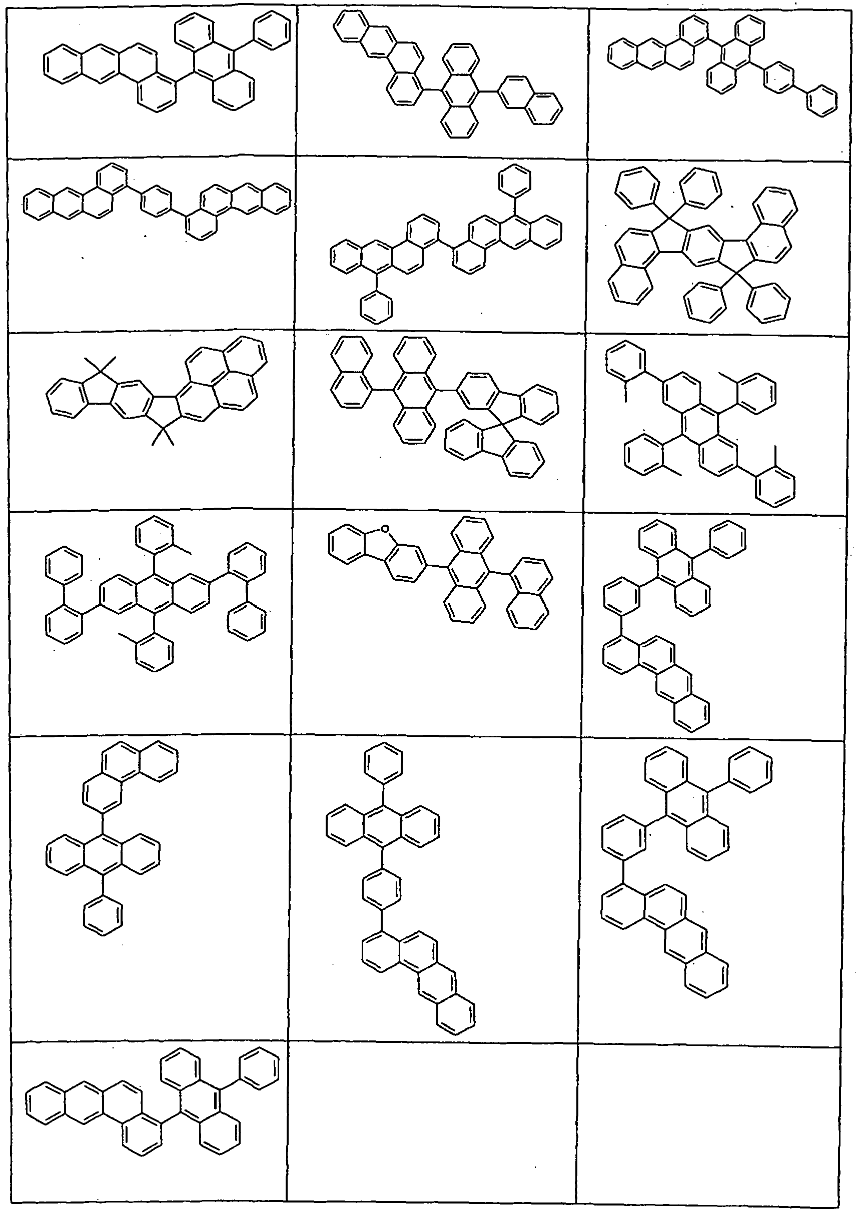

Geeignete Ladungstransportmaterialien, wie sie in der Lochinjektionsbzw. Lochtransportschicht oder in der Elektronentransportschicht der erfindungsgemäßen organischen Elektrolumineszenzvorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Kathode der organischen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Ba/Ag oder Mg/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch MetalliMetalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink-Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Valcuum-Sublimationsanfagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I), (II) oder (III) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Die organischen Elektrolumineszenzvorrichtungen enthaltend eine oder mehrere erfindungsgemäße Verbindungen können in Displays, als Lichtquellen in Beleuchtungsanwendungen sowie als Lichtquellen in medizinischen und/oder kosmetischen Anwendungen (z.B. Lichttherapie) eingesetzt werden.

Bei Verwendung der Verbindungen gemäß Formel (I), (II) oder (III) in einer organischen Elektrolumineszenzvorrichtung können einer oder mehrere der im Folgenden genannten Vorteile realisiert werden:
Die erfindungsgemäßen Verbindungen eignen sich sehr gut zur Verwendung als Matrixmaterialien für phosphoreszierende Dotanden sowie zur Verwendung als Elektronentransportmaterialien. Bei Verwendung der erfindungsgemäßen Verbindungen in diesen Funktionen werden gute Leistungseffizienzen, geringe Betriebsspannungen und gute Lebensdauern der organischen Elektrolumineszenzvorrichtungen erhalten.

Weiterhin zeichnen sich die erfindungsgemäßen Verbindungen durch eine hohe Oxidationsstabilität in Lösung aus, was sich vorteilhaft bei der Aufreinigung und Handhabung der Verbindungen sowie bei ihrer Verwendung in elektronischen Vorrichtungen auswirkt.

Ferner sind die erfindungsgemäßen Verbindungen temperaturstabil und können somit weitgehend zersetzungsfrei sublimiert werden. Die Aufreinigung der Verbindungen wird dadurch erleichtert, und die Verbindungen können in höherer Reinheit erhalten werden, was sich positiv auf die Leistungsdaten der elektronischen Vorrichtungen enthaltend die Materialien auswirkt. Insbesondere können dadurch Vorrichtungen mit längeren operativen Lebensdauern hergestellt werden.

Die Erfindung wird durch die nachfolgenden Ausführungsbeispiele näher erläutert.

### Ausführungsbeispiele

### A) Synthesebeispiele

Die nachfolgenden Synthesen werden, sofern nicht anders angegeben, unter einer Schutzgasatmosphäre in getrockneten Lösungsmitteln durchgeführt. Als Edukte bzw. Reaktanden können z.B. Pyrrolo[3,4-b]indol-3,4(2H)-dicarbonsäure-3,4-dimethylester (Science of Synthesis 2002, 9,441-552), 2-(3-Bromphenyl)-4,6-diphenyl-pyrimidin, 4-(3-Bromphenyl)-4,6-diphenyl-pyrimidin, 4-(3,5-Dibromphenyl)-4,6-diphenyl-pyrimidin (WO 2005/085387) und 2-(3,5-Dibromphenyl)-4,6-diphenyl-pyrimidin eingesetzt werden.

### Beispielverbindung 1

### a) 2-Phenyl-2H-pyrrolo[3,4-b]indol-3,4-dicarbonsäurediethylester

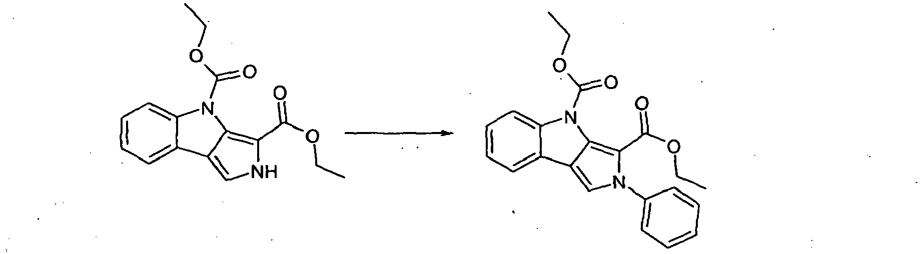

53 g (176 mmol) Pyrrolo[3,4-b]indol-3,4(2H)-dicarbonsäurediethylester und 129 g (1059 mmol) Phenylboronsäure werden in 2000 mL Dichloromethan gelöst und entgast. Es wird mit 71 mL Triarylamin und mit 40 g (356 mmol) Kupfer(II)acetat und 111 g Molekularsieb (0.4 NM) versetzt. Die Reaktionsmischung wird anschließend bei Raumtemperatur für 80 h unter Schutzgasatmosphäre gerührt. Die abgekühlte Lösung wird mit Toluol verdünnt, mehrmals mit Wasser gewaschen, getrocknet und eingeengt. Das Produkt wird via Säulenchromatographie an Kieselgel mit Toluol/Heptan (1:2) gereinigt. Ausbeute: 55.4 g (141 mmol), 80 % der Theorie.

### b) 2-Phenyl-2,4-dihydro-pyrrolo[3,4-b]indol-3-carbonsäureethylester

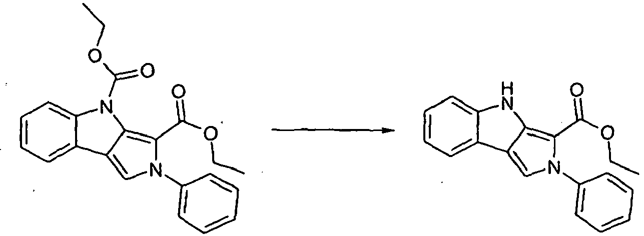

37,6 g (100 mmol)2-Phenyl-2H-pyrrolo[3,4-b]indol-3,4-dicarbonsäurediethylester werden mit einer Lösung von 41g (300 mmol) K₂CO₃ in 1500 mL MeOH-H2O (3:1) versetzt und 2h unter Rückfluss erhitzt. Nacht dem Abkühlen wird mit Dichloromethan extrahiert und nach Phasentrennung getrocknet und eingeengt. Das Produkt wird via Säulenchromatographie an Kieselgel mit Toluol/Heptan (1:2) gereinigt. Ausbeute: 19.7 g (65 mmol), 65 % der Theorie.

### c) 2-(2-Phenyl-2,4-dihydro-pyrrolo[3,4-b]indol-3-yl)-propan-2-ol

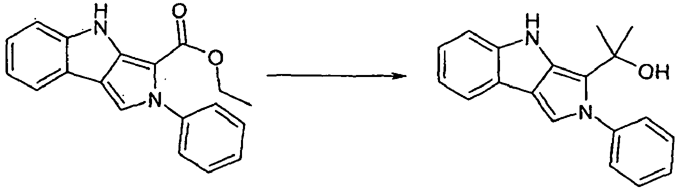

64.7 g (213 mmol) 2-Phenyl-2,4-dihydro-pyrrolo[3,4-b]indol-3-carbonsäureethylester werden in 1500 mL getrocknetem THF gelöst und entgast. Es wird auf -78 °C gekühlt und innerhalb von 40 min mit 569 ml (854 mmol) Methyllithium versetzt. Man lässt innerhalb 1 h bis auf -40 °C erwärmen und kontrolliert die Umsetzung via DC. Nach vollständiger Umsetzung wird bei -30 °C vorsichtig mit MeOH gequencht. Die Reaktionslösung wird auf 1/3 eingeengt und mit 1 L Methylenchlorid versetzt und gewaschen. Die organische Phase wird über MgSO₄ getrocknet und eingeengt. Ausbeute: 55.5 g (191 mmol), 90 % d. Th., Reinheit nach ¹H-NMR ca. 94 %.

### d) Zwischenstufe d)

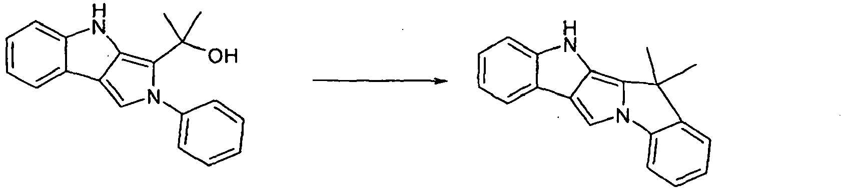

12.6 g (43.6 mmol) 2-(2-Phenyl-2,4-dihydro-pyrrolo[3,4-b]indo(-3-yl)-propan-2-ol werden in 1200 mL entgastem Toluol gelöst und mit einer Suspension aus 40 g Polyphosphorsäure und 28 mL Methansulfonsäure versetzt und für 1 h auf 60 °C erhitzt. Der Ansatz wird abgekühlt und mit Wasser versetzt. Es fällt ein Feststoff aus, der mit Methylenchlorid/THF (1:1) gelöst wird. Die Lösung wird mit 20%iger NaOH vorsichtig alkalisiert, die Phasen werden getrennt und über MgSO₄ getrocknet. Der erhaltene Feststoff wird aus Heptan ausgerührt. Ausbeute: 10.8 g (39 mmol), 92% d. Th., Reinheit nach ¹H-NMR ca. 98 %.

### e) Beispielverbindung 1

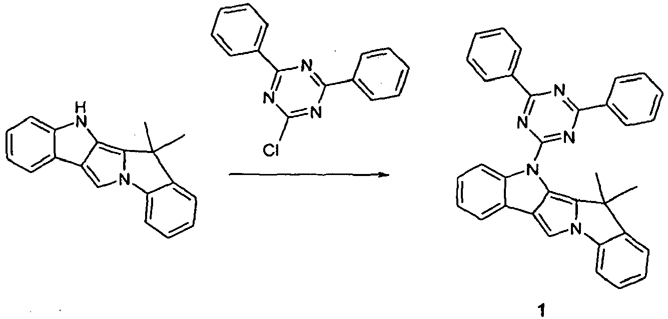

68 g (250 mmol) der Zwischenstufe d) werden in 1000 mL Dimethylformamid unter Schutzatmosphäre gelöst und mit 13.8 g NaH 60%ig in Mineralöl (345 mmol) versetzt. Nach 1 Stunde bei Raumtemperatur wird eine Lösung von 73 g (270 mmol) 2-Chlor-4,6-Diphenyl-[1,3,5]triazin in 1000 mL THF zugetropft. Das Reaktionsgemisch wird anschließend 12 h bei Raumtemperatur gerührt. Nach dieser Zeit wird das Reaktionsgemisch auf Eis gegossen und dreimal mit Dichlormethan extrahiert. Die vereinigten organischen Phasen werden über Na₂SO₄ getrocknet und eingeengt. Der Rückstand wird mit Toluol heiß extrahiert und aus Toluol umkristallisiert und abschließend im Hochvakuum sublimiert. Die Reinheit beträgt 99.9%, Ausbeute 99 g (79 %).

Nach analogen Synthesevorschriften werden die folgenden Verbindungen erhalten:

| **Bsp.** | **Edukt 1** | **Edukt 2** | **Produkt** | **Ausbeute** |
|---|---|---|---|---|
| 1b | 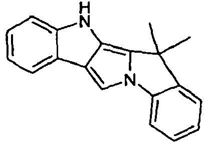 | 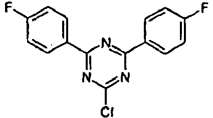 | 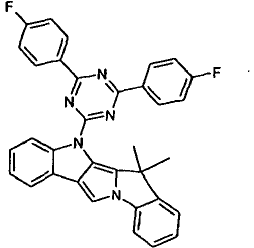 | 65% |
| | | **804548-71-8** | | |
| 1c | 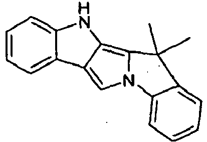 | 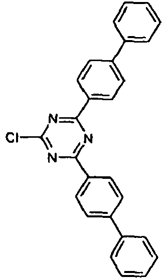 | 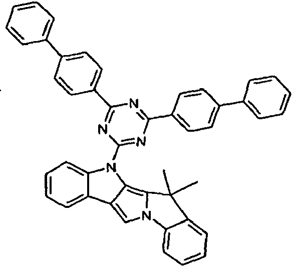 | 77% |
| | | **182918-13-4** | | |
| 1d | 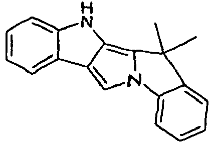 | 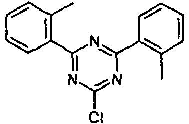 | 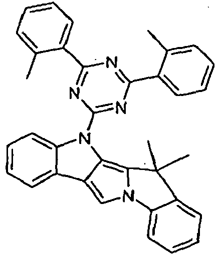 | 63% |
| | | **78941-34-1** | | |

### Synthese der Zwischenstufe 4-(2-Brom-phenyl)-2,6-diphenylpyrimidin

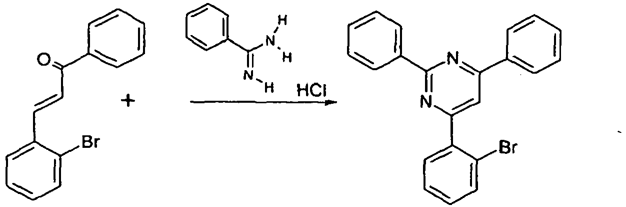

23 g (409 mmol) Kaliumhydroxid werden in 500 mL Ethanol gelöst, bei Raumtemperatur mit 40g (255 mmol) Benzamid-hydrochlorid und 129 g (452 mmol) (3-(Bromphenyl)-1-phenyl-2-propen-1-on gelöst, mit 500 ml Ethanol versetzt und 3 h unter Rückfluss gerührt. Nach Kühlung auf Raumtemperatur wird der ausgefallene Feststoff abgesaugt, mit etwas EtOH gewaschen und getrocknet. Es verbleiben 55 g (129 mmol), 50 % des Produkts 4-(2-Brom-phenyl)-2,6-diphenyl-pyrimidin in Form farbloser Kristalle.

### Beispielverbindung 2

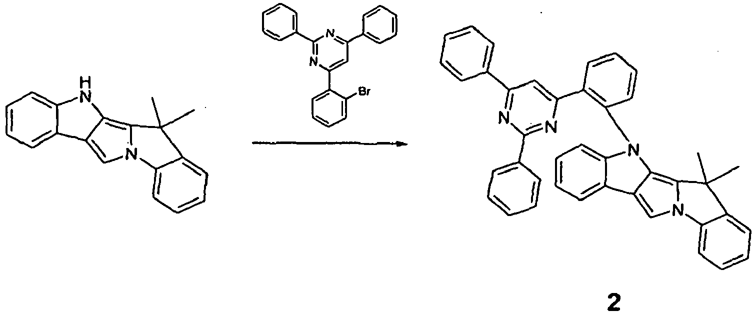

Unter Schutzgas werden 21 g (79.8 mmol) 2,12-Dimethyl-10-phenyl-10,12-dihydro-10-aza-indeno[2,1-b]fluoren, 34 g (87 mmol) 4-(2-Bromphenyl)-2,6-diphenyl-pyrimidin, 15.9 ml (15.9 mmol) 1 mol/L Tri-tert-butylphosphin und 1.79 g (7.9 mmol) Palladiumacetat in 120 ml p-Xylol suspendiert. Die Reaktionsmischung wird 16 h unter Rückfluss erhitzt. Nach Erkalten wird die organische Phase abgetrennt, dreimal mit 200 mL Wasser gewaschen und anschließend zur Trockene eingeengt. Der Rückstand wird mit Toluol heiß extrahiert, aus Toluol umkristallisiert und abschließend im Hochvakuum sublimiert. Die Reinheit beträgt 99.9%, Ausbeute 36 g (62 mmol), 81 % der Theorie.

Nach analoger Synthesevorschrift werden die folgenden Verbindungen erhalten:

| **Bsp.** | **Edukt 1** | **Edukt 2** | **Produkt** | **Ausbeute** |
|---|---|---|---|---|
| 2b | 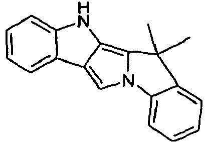 | 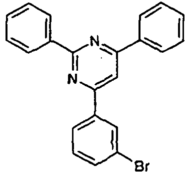 | 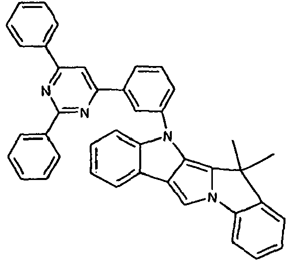 | 70% |
| | | 864377-28-6 | | |
| 2c | 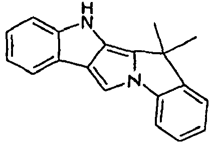 | 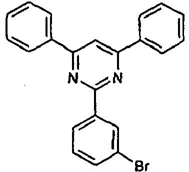 | 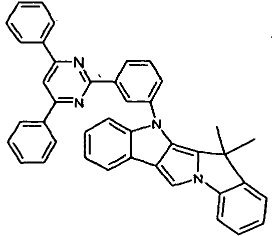 | 76% |
| | | 864377-22-0 | | |
| 2d | 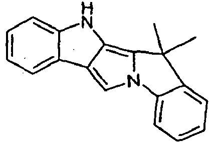 | 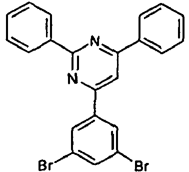 | 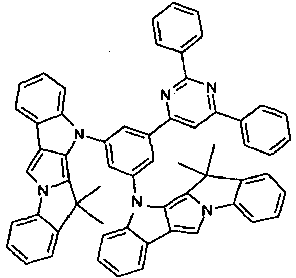 | 64% |
| | | 607740-08-9 | | |
| 2e | 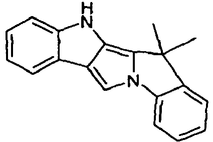 | 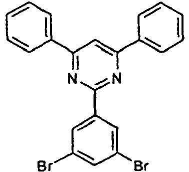 | 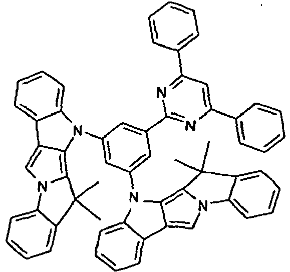 | 71% |
| | | 942132-67-4 | | |

### B) Device-Beispiele: Herstellung der OLEDs

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, Materialien) angepasst wird.

In den folgenden Beispielen E1-E8 (siehe Tabellen 1 und 2) werden die Daten verschiedener OLEDs vorgestellt. Glasplättchen, die mit strukturiertem ITO (Indium Zinn Oxid) der Dicke 150 nm beschichtet sind, werden zur verbesserten Prozessierung mit 20 nm PEDOT beschichtet (Poly(3,4-ethylendioxy-2,5-thiophen), aus Wasser aufgeschleudert, bezogen von H. C. Starck, Goslar, Deutschland). Diese beschichteten Glasplättchen bilden die Substrate, auf welche die OLEDs aufgebracht werden. Die OLEDs haben prinzipiell folgenden Schichtaufbau: Substrat / Lochtransportschicht (HTL) / Optionale Zwischenschicht (IL) / Elektronenblockerschicht (EBL) / Emissionsschicht (EML) / Optionale Lochblockierschicht (HBL) / Elektronentransportschicht (ETL) / Optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrix-material (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H5:TER2 (88%:12%) bedeutet hierbei, dass das Material H5 in einem Volumenanteil von 88% und TER2 in einem Anteil von 12% in der Schicht vorliegt.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m².

Die Daten der verschiedenen OLEDs sind in Tabelle 2 zusammengefasst. Die erfindungsgemäßen Materialien ergeben bei Einsatz als Matrixmaterialien für rot und grün phosphoreszierenden Emitter gute Effizienz und Betriebsspannung (Beispiele E1-E7 in Tabelle 2). Weiterhin ergeben sich bei Einsatz des Materials H3 als Elektronentransportmaterial gute Leistungsdaten (Beispiel E8).

**Tabelle 1: Aufbau der OLEDs**

| Bsp. | HTL | IL | EBL | EML | HBL | ETL | EIL |
|---|---|---|---|---|---|---|---|
| | Dicke | Dicke | Dicke | Dicke | Dicke | Dicke | Dicke |
| E1 | SpA1 | HATCN | BPA1 | H1:TEG1 (90%:10%) | --- | ST1:LiQ (50%:50%) | --- |
| | 70nm | 5nm | 90nm | 30nm | | 40 nm | |
| E2 | SpA1 | --- | NPB | H2:TER1 (85%:15%) | --- | Alq₃ | LiF |
| | 20nm | | 20nm | 30nm | | 20nm | 1nm |
| E3 | SpA1 | HATCN | BPA1 | H3:TEG1 (90%:10%) | --- | ST1:LiQ (50%:50%) | --- |
| | 70nm | 5nm | 90nm | 30nu | | 40 nm | |
| E4 | SpA1 | HATCN | BPA1 | H4:TEG1 (90%:10%) | IC1 | ST1:LiQ (50%:50%) | --- |
| | 70nm | 5nm | 90nm | 30nm | 10nm | 40 nm | |
| E5 | SpA1 | HATCN | SpA2 | H5:TEG1 (90%:10%) | IC1 | ST1:LiQ (50%:50%) | --- |
| | 70nm | 5nm | 90nm | 30nm | 10nm | 40 nm | |
| E6 | SpA1 | --- | NPB | H5:TER2 (88%:12%) | ST1 | ST1:LiQ (50%:50%) | LiQ |
| | 20nm | | 20nm | 30nm | 5nm | 20nm | 1nm |
| E7 | SpA1 | HATCN | BPA1 | H6:TEG1 (90%:10%) | IC1 | ST1:LiQ (50%:50%) | --- |
| | 70nm | 5nm | 90nm | 30nm | 10nm | 40 nm | |
| E8 | SpA1 | HATCN | BPA1 | IC1:TEG1 (90%:10%) | --- | H3 | LiQ |
| | 70nm | 5nm | 90nm | 30nm | | 40 nm | 3nm |

**Tabelle 2: Daten der OLEDS**

| Bsp. | U1000 | SE1000 | LE1000 | EQE | CIE x/y bei |
|---|---|---|---|---|---|
| | (V) | (cd/A) | (lm/W) | 1000 | 1000 cd/m² |
| E1 | 3.6 | 47 | 41 | 13.1% | 0.36/0.60 |
| E2 | 5.2 | 5.8 | 3.5 | 9.6% | 0.69/0.31 |
| E3 | 3.8 | 38 | 31 | 10.4% | 0.36/0.60 |
| E4 | 3.9 | 42 | 34 | 11.7% | 0.37/0.60 |
| E5 | 3.4 | 52 | 49 | 14.5% | 0.36/0.60 |
| E6 | 5.4 | 10.7 | 6.2 | 9.9% | 0.66/0.33 |
| E7 | 3.6 | 43 | 38 | 11.9% | 0.36/0.61 |
| E8 | 3.8 | 48 | 40 | 13.4% | 0.36/0.60 |

**Tabelle 3: Strukturformeln der Materialien für die OLEDs**

| | |
|---|---|
| 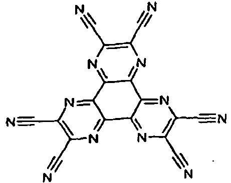 | 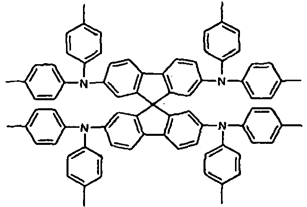 |
| HATCN | SpA1 |
| 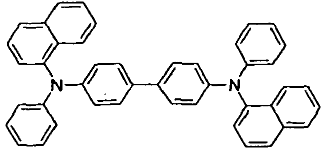 | 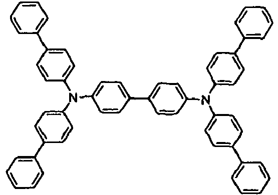 |
| NPB | BPA1 |
| 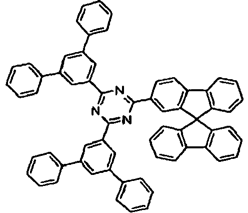 | 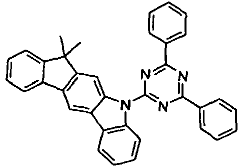 |
| ST1 | IC1 |
| 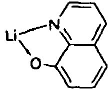 | 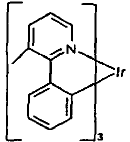 |
| LiQ | TEG1 |
| 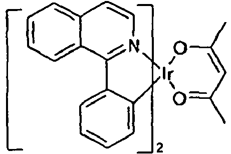 | 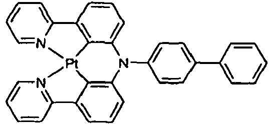 |
| TER1 | TER2 |
| 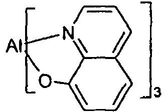 | 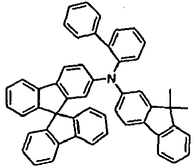 |
| Alg₃ | SpA2 |
| 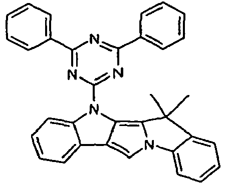 | 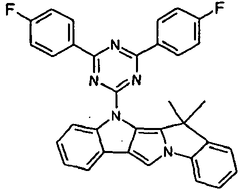 |
| H1 | H2 |
| (Beispielverbindung **1**) | (Beispielverbindung **1b**) |
| 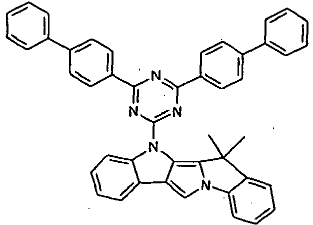 | 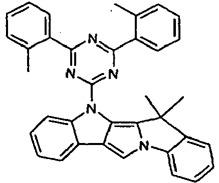 |
| H3 | H4 |
| (Beispielverbindung **1c**) | (Beispielverbindung **1d)** |
| 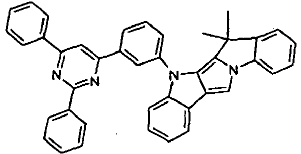 | 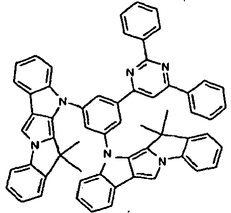 |
| H5 | H6 |
| (Beispielverbindung **2b**) | (Beispielverbindung **2d**) |

## Patentansprüche

1. Organische Elektrolumineszenzvorrichtung, **dadurch gekennzeichnet, dass** eine Verbindung der Formel (I), (II) oder (III) 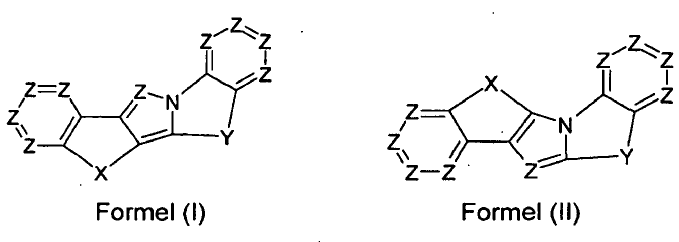 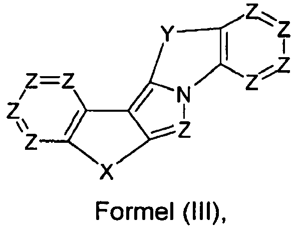 wobei für die auftretenden Symbole gilt:
Z ist bei jedem Auftreten gleich oder verschieden ausgewählt aus CR² und N;
X, Y sind bei jedem Auftreten gleich oder verschieden eine divalente Gruppe, ausgewählt aus BR², C(R²)₂, C=O, C=NR², C=S, Si(R²)₂, NR¹, PR², P(=O)R², O, S, S=O und S(=O)₂;
R¹ ist bei jedem Auftreten gleich oder verschieden ein aromatisches oder heteroaromatisches Ringsystem mit 5 bis 60 aromatischen Ringatomen, welches mit einem oder mehreren Resten R³ substituiert sein kann und welches mit einem Substituenten R² am Grundgerüst oder einem Atom des Grundgerüsts verknüpft sein kann;
R² ist bei jedem Auftreten gleich oder verschieden H, D, F, Cl, Br, I, B(OR³)₂, CHO, C(=O)R³, CR³=C(R³)₂, CN, C(=O)OR³, C(=O)N(R³)₂, Si(R³)₃, N(R³)₂, N(Ar¹)2, NO₂, P(=O)(R³)₂, OSO₂R³, OR³, S(=O)R³, S(=O)₂R³, eine geradkettige Alkyl-, Alkoxy- oder Thioalkylgruppe mit 1 bis 20 C-Atomeri oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere benachbarte oder nicht benachbarte CH₂-Gruppen in den oben genannten Gruppen durch -R³C=C_{R}³-, -C=C-, Si(R³)₂, Ge(R³)₂, Sn(R³)₂, C=O, C=S, C=Se,
C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R³ substituiert sein kann, oder eine Aryloxy- oder Heteroaryloxygruppe mit 5 bis 60 aromatischen Ringatomen, die durch einen oder mehrere Reste R³ substituiert sein kann, wobei zwei oder mehr Reste R² miteinander verknüpft sein können und einen Ring oder ein Ringsystem bilden können;
R³ ist bei jedem Auftreten gleich oder verschieden H, D, F, Cl, Br, I, B(OR⁴)₂, CHO, C(=O)_{R}⁴, CR⁴=C(R⁴)₂, CN, C(=O)OR⁴, C(=O)N(R⁴)₂, Si(R⁴)₃, N(R⁴)₂, NO₂, P(=O)(R⁴)₂, OSO₂R⁴, OR⁴, S(=O)R⁴, S(=O)₂R⁴, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere benachbarte oder nicht benachbarte CH₂-Gruppen in den oben genannten Gruppen durch - R⁴C=CR⁴-, -C=C-, Si(R⁴)₂, Ge(R⁴)₂, Sn(R⁴)₂, C=O, C=S, C=Se, C=NR⁴, -C(=O)O-, -C(=O)NR⁴-, NR⁴, P(=O)(R⁴), -O-, - S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R⁴ substituiert sein kann, oder eine Aryloxy- oder Heteroaryloxygruppe mit 5 bis 60 aromatischen Ringatomen, die durch einen oder mehrere Reste R⁴ substituiert sein kann, wobei zwei oder mehr Reste R³ miteinander verknüpft sein können und einen Ring oder ein Ringsystem bilden können;
R⁴ ist gleich oder verschieden bei jedem Auftreten H, D, F oder ein aliphatischer, aromatischer und/oder heteroaromatischer organischer Rest mit 1 bis 20 C-Atomen, in dem auch ein oder mehrere H-Atome durch D oder F ersetzt sein können; dabei können zwei oder mehr Substituenten R⁴ auch miteinander verknüpft sein und einen Ring oder ein Ringsystem bilden;
Ar¹ ist bei jedem Auftreten gleich oder verschieden ein aromatisches oder heteroaromatisches Ringsystem mit 5 bis 30 aromatischen Ringatomen, das mit einem oder mehreren Resten R³ substituiert sein kann;
oder ein Polymer, Oligomer oder Dendrimer, enthaltend eine oder mehrere Verbindungen der Formel (I), (II) oder (III), wobei die Bindung(en) zum Polymer, Oligomer oder Dendrimer an beliebigen, in Formel (I), (II) bzw. (III) mit R¹ oder R² substituierten Positionen lokalisiert sein können,
als Lochtransportmaterial in einer Lochtransportschicht oder Lochinjektionsschicht eingesetzt wird und/oder als Matrixmaterial in einer emittierenden Schicht eingesetzt wird und/oder als Elektronentransportmaterial in einer Elektronentransportschicht eingesetzt wird.

2. Organische Elektrolumineszenzvorrichtung nach Anspruch 1, **dadurch gekennzeichnet, dass** 0, 1 oder 2 Gruppen Z pro aromatischem Sechsring einer Verbindung der Formel (I), (II) oder (III) gleich N sind, und die restlichen Gruppen Z gleich CR² sind.

3. Organische Elektrolumineszenzvorrichtung nach Anspruch 1 oder 2, **dadurch gekennzeichnet, dass** X ausgewählt ist aus C=O, NR¹, O, S, S=O und S(=O)₂.

4. Organische Elektrolumineszenzvorrichtung nach einem oder mehreren der Ansprüche 1 bis 3, **dadurch gekennzeichnet, dass** Y ausgewählt ist aus C(R²)₂, NR¹, O und S.

5. Organische Elektrolumineszenzvorrichtung nach einem oder mehreren der Ansprüche 1 bis 4, **dadurch gekennzeichnet, dass** R² bei jedem Auftreten gleich oder verschieden ausgewählt ist aus H, D, F, CN, Si(R³)₃, N(R³)₂ oder einer geradkettigen Alkyl- oder Alkoxygruppe mit 1 bis 20 C-Atomen oder einer verzweigten oder cyclischen Alkyl- oder Alkoxygruppe mit 3 bis 20 C-Atomen, wobei die oben genannten Gruppen jeweils mit einem oder mehreren Resten R³ substituiert sein können und wobei in den oben genannten Gruppen eine oder mehrere benachbarte oder nicht benachbarte CH₂-Gruppen durch -C≡C-, -R³C=CR³-, Si(R³)₂, C=O, C=NR³, -NR³-, -O-, -S-, - C(=O)O- oder -C(=O)NR³- ersetzt sein können, oder ein aromatisches oder heteroaromatisches Ringsystem mit 5 bis 20 aromatischen Ringatomen, das jeweils mit einem oder mehreren Resten R³ substituiert sein kann.

6. Organische Elektrolumineszenzvorrichtung nach einem oder mehreren der Ansprüche 1 bis 5, **dadurch gekennzeichnet, dass** mindestens eine Gruppe R¹ oder R² vorhanden ist, welche ausgewählt ist aus
- Heteroarylgruppen mit 1 bis 20 C-Atomen, welche wahlweise über eine oder mehrere dazwischen gebundene divalente Arylgruppen gebunden sein können und welche mit einem oder mehreren Resten R³ substituiert sein können,
- aromatischen oder heteroaromatischen Ringsystemen mit 5 bis 20 aromatischen Ringatomen, welche mit einem oder mehreren Resten R³ substituiert sein können, und
- Arylamingruppen, welche mit einem oder mehreren Resten R³ substituiert sein können.

7. Organische Elektrolumineszenzvorrichtung nach Anspruch 6, **dadurch gekennzeichnet, dass** die Heteroarylgruppen ausgewählt sind aus Pyridin, Pyrimidin, Pyridazin, Pyrazin, Triazin und Benzimidazol, welche mit einem oder mehreren Resten R³ substituiert sein können, und die aromatischen oder heteroaromatischen Ringsysteme ausgewählt sind aus Naphthyl, Anthracenyl, Phenanthrenyl, Benzanthracenyl, Pyrenyl, Biphenyl, Terphenyl und Quaterphenyl, welche mit einem oder mehreren Resten R³ substituiert sein können.

8. Organische Elektrolumineszenzvorrichtung nach einem oder mehreren der Ansprüche 1 bis 7, **dadurch gekennzeichnet, dass** R¹ bei jedem Auftreten gleich oder verschieden ausgewählt ist aus einem aromatischen oder heteroaromatischen Ringsystem mit 5 bis 30 aromatischen Ringatomen, welches mit einem oder mehreren Resten R³ substituiert sein kann.

9. Organische Elektrolumineszenzvorrichtung nach einem oder mehreren der Ansprüche 1 bis 8, **dadurch gekennzeichnet, dass** R³ bei jedem Auftreten gleich oder verschieden ausgewählt ist aus H, D, F, CN, Si(R⁴)₃, N(R⁴)₂ oder einer geradkettigen Alkyl- oder Alkoxygruppe mit 1 bis 20 C-Atomen oder einer verzweigten oder cyclischen Alkyl- oder Alkoxygruppe mit 3 bis 20 C-Atomen, wobei die oben genannten Gruppen jeweils mit einem oder mehreren Resten R⁴ substituiert sein können und wobei in den oben genannten Gruppen eine oder mehrere benachbarte oder nicht benachbarte CH₂-Gruppen durch -C=C-, -R⁴C=CR⁴-, Si(R⁴)₂, C=O, C=NR⁴, -NR⁴-, -O-, -S-, - C(=O)O- oder -C(=O)NR⁴- ersetzt sein können, oder einem aromatischen oder heteroaromatischen Ringsystem mit 5 bis 20 aromatischen Ringatomen, das jeweils mit einem oder mehreren Resten R⁴ substituiert sein kann.

10. Organische Elektrolumineszenzvorrichtung nach einem oder mehreren der Ansprüche 1 bis 9, **dadurch gekennzeichnet, dass** R⁴ bei jedem Auftreten gleich oder verschieden gewählt ist aus H, D, F, oder einem aliphatischen, aromatischen und/oder heteroaromatischen organischen Rest mit 1 bis 20 C-Atomen, in dem auch ein oder mehrere H-Atome durch D oder F ersetzt sein können.

## Claims

1. Organic electroluminescent device, **characterised in that** a compound of the formula (I), (II) or (III) 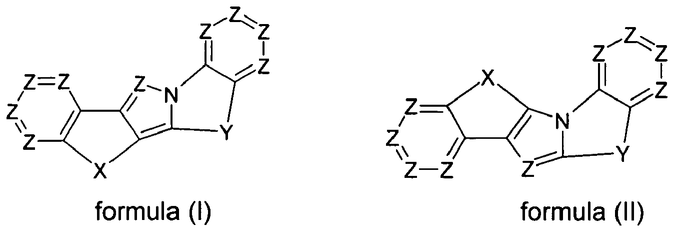 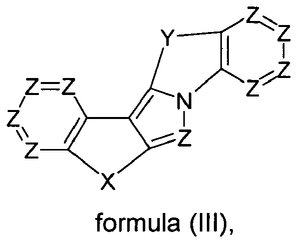 where the following applies to the symbols occurring:
Z is selected on each occurrence, identically or differently, from CR² and N;
X, Y are on each occurrence, identically or differently, a divalent group selected from BR², C(R²)₂, C=O, C=NR², C=S, Si(R²)₂, NR¹, PR², P(=O)R², O, S, S=O and S(=O)₂;
R¹ is on each occurrence, identically or differently, an aromatic or heteroaromatic ring system having 5 to 60 aromatic ring atoms, which may be substituted by one or more radicals R³ and which may be linked to a substituent R² on the skeleton or an atom of the skeleton;
R² is on each occurrence, identically or differently, H, D, F, Cl, Br, I, B(OR³)₂, CHO, C(=O)_{R}³, CR³=C(R³)₂, CN, C(=O)OR³, C(=O)N(R³)₂, Si(R³)₃, N(R³)₂, N(Ar¹)₂, NO₂, P(=O)(R³)₂, OSO₂R³, OR³, S(=O)R³, S(=O)₂R³, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R³ and where one or more adjacent or non-adjacent CH₂ groups in the above-mentioned groups may be replaced by -R³C=CR³-, -C=C-, Si(R³)₂, Ge(R³)₂, Sn(R³)₂, C=O, C=S, C=Se, C=NR³, -C(=O)O-, -C(=O)NR³-, NR³, P(=O)(R³), -O-, -S-, SO or SO₂ and where one or more H atoms in the above-mentioned groups may be replaced by D, F, Cl, Br, I, CN or NO₂, or an aromatic or heteroaromatic ring system having 5 to 60 aromatic ring atoms, which may in each case be substituted by one or more radicals R³, or an aryloxy or heteroaryloxy group having 5 to 60 aromatic ring atoms, which may be substituted by one or more radicals R³, where two or more radicals R² may be linked to one another and may form a ring or ring system;
R³ is on each occurrence, identically or differently, H, D, F, Cl, Br, I, B(OR⁴)₂, CHO, C(=O)R⁴, CR⁴-C(R⁴)_{2,} CN, C(=O)OR⁴, C(=O)N(R⁴)₂, Si(R⁴)₃, N(R⁴)₂, NO₂, P(=O)(R⁴)₂, OSO₂R⁴, OR⁴, S(=O)R⁴, S(=O)₂R⁴, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R⁴ and where one or more adjacent or non-adjacent CH₂ groups in the above-mentioned groups may be replaced by -R⁴C=CR⁴-, C≡C-, Si(R⁴)₂, Ge(R⁴)₂, Sn(R⁴)₂, C=O, C=S, C=Se, C=NR⁴, -C(=O)O-, -C(=O)NR⁴-, NR⁴, P(=O)(R⁴), -O-, -S-, SO or SO₂ and where one or more H atoms in the above-mentioned groups may be replaced by D, F, Cl, Br, 1, CN or NO₂, or an aromatic or heteroaromatic ring system having 5 to 60 aromatic ring atoms, which may in each case be substituted by one or more radicals R⁴, or an aryloxy or heteroaryloxy group having 5 to 60 aromatic ring atoms, which may be substituted by one or more radicals R⁴, where two or more radicals R³ may be linked to one another and may form a ring or ring system;
R⁴ is, identically or differently on each occurrence, H, D, F or an aliphatic, aromatic and/or heteroaromatic organic radical having 1 to 20 C atoms, in which, in addition, one or more H atoms may be replaced by D or F; two or more substituents R⁴ here may also be linked to one another and form a ring or ring system;
Ar¹ is on each occurrence, identically or differently, an aromatic or heteroaromatic ring system having 5 to 30 aromatic ring atoms, which may be substituted by one or more radicals R³;
or a polymer, oligomer or dendrimer containing one or more compounds of the formula (I), (II) or (III), where the bond(s) to the polymer, oligomer or dendrimer may be localised at any desired positions in formula (I), (II) or (III) that are substituted by R¹ or R²,
is employed as hole-transport material in a hole-transport layer or hole-injection layer and/or is employed as matrix material in an emitting layer and/or is employed as electron-transport material in an electron-transport layer.

2. Organic electroluminescent device according to Claim 1, **characterised in that** 0, 1 or 2 groups Z per aromatic six-membered ring of a compound of the formula (I), (II) or (III) are equal to N, and the remaining groups Z are equal to CR².

3. Organic electroluminescent device according to Claim 1 or 2, **characterised in that** X is selected from C=O, NR¹, O, S, S=O and S(=O)₂.

4. Organic electroluminescent device according to one or more of Claims 1 to 3, **characterised in that** Y is selected from C(R²)₂, NR¹, O and S.

5. Organic electroluminescent device according to one or more of Claims 1 to 4, **characterised in that** R² is selected on each occurrence, identically or differently, from H, D, F, CN, Si(R³)₃, N(R³)₂ or a straight-chain alkyl or alkoxy group having 1 to 20 C atoms or a branched or cyclic alkyl or alkoxy group having 3 to 20 C atoms, where the above-mentioned groups may each be substituted by one or more radicals R³ and where one or more adjacent or non-adjacent CH₂ groups in the above-mentioned groups may be replaced by -C=C-, -R³C=CR³-, Si(R³)₂, C=O, C=NR³, -NR³-, -O-, -S-, -C(=O)O- or -C(=O)NR³-, or an aromatic or heteroaromatic ring system having 5 to 20 aromatic ring atoms, which may in each case be substituted by one or more radicals R³

6. Organic electroluminescent device according to one or more of Claims 1 to 5, **characterised in that** at least one group R¹ or R² is present which is selected from
- heteroaryl groups having 1 to 20 C atoms, which may optionally be bonded via one or more divalent aryl groups bonded in between and which may be substituted by one or more radicals R³,
- aromatic or heteroaromatic ring systems having 5 to 20 aromatic ring atoms, which may be substituted by one or more radicals R³, and
- arylamine groups, which may be substituted by one or more radicals R³.

7. Organic electroluminescent device according to Claim 6, **characterised in that** the heteroaryl groups are selected from pyridine, pyrimidine, pyridazine, pyrazine, triazine and benzimidazole, each of which may be substituted by one or more radicals R³, and the aromatic or heteroaromatic ring systems are selected from naphthyl, anthracenyl, phenanthrenyl, benzanthracenyl, pyrenyl, biphenyl, terphenyl and quaterphenyl, each of which may be substituted by one or more radicals R³.

8. Organic electroluminescent device according to one or more of Claims 1 to 7, **characterised in that** R¹ is selected on each occurrence, identically or differently, from an aromatic or heteroaromatic ring system having 5 to 30 aromatic ring atoms, which may be substituted by one or more radicals R³.

9. Organic electroluminescent device according to one or more of Claims 1 to 8, **characterised in that** R³ is selected on each occurrence, identically or differently, from H, D, F, CN, Si(R⁴)₃, N(R⁴)₂ or a straight-chain alkyl or alkoxy group having 1 to 20 C atoms or a branched or cyclic alkyl or alkoxy group having 3 to 20 C atoms, where the above-mentioned groups may each be substituted by one or more radicals R⁴ and where one or more adjacent or non-adjacent CH₂ groups in the above-mentioned groups may be replaced by -C≡C-, -R⁴C=CR⁴-, Si(R⁴)₂, C=O, C=NR⁴, -NR⁴-, -O-, -S-, -C(=O)O- or -C(=O)NR⁴-, or an aromatic or heteroaromatic ring system having 5 to 20 aromatic ring atoms, which may in each case be substituted by one or more radicals R⁴.

10. Organic electroluminescent device according to one or more of Claims 1 to 9, **characterised in that** R⁴ is selected on each occurrence, identically or differently, from H, D, F, or an aliphatic, aromatic and/or heteroaromatic organic radical having 1 to 20 C atoms, in which, in addition, one or more H atoms may be replaced by D or F.

## Revendications

1. Dispositif électroluminescent organique, **caractérisé en ce qu'**un composé de la formule (I), (II) ou (III) : 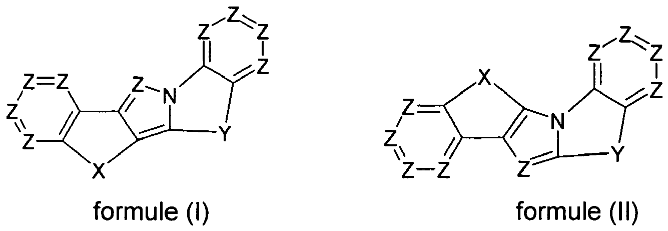 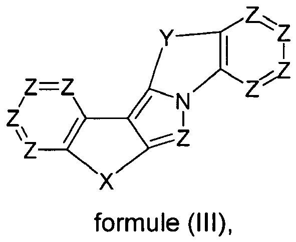 dans lesquelles ce qui suit s'applique aux symboles rencontrés :
Z est choisi, pour chaque occurrence, de manière identique ou différente, parmi CR² et N ;
X, Y sont, pour chaque occurrence, de manière identique ou différente, un groupe divalent choisi parmi BR², C(R²)₂, C=O, C=NR², C=S, Si(R²)₂, NR¹, PR², P(=O)R², O, S, S=O et S(=O)₂ ;
R¹ est, pour chaque occurrence, de manière identique ou différente, un système de cycle aromatique ou hétéroaromatique comportant 5 à 60 atomes de cycle aromatique, lequel peut être substitué par un radical ou plusieurs radicaux R³ et lequel peut être lié à un substituant R² sur le squelette ou sur un atome du squelette ;
R² est, pour chaque occurrence, de manière identique ou différente, H, D, F, CI, Br, 1, B(OR³)₂, CHO, C(=O)R³, CR³=C(R³)₂, CN, C(=O)OR³, C(=O)N(R³)₂, Si(R³)₃, N(R³)₂, N(Ar¹)₂, NO₂, P(=O)(R³)₂, OSO₂R³, OR³, S(=O)R³, S(=O)₂R³, un groupe alkyle, alcoxy ou thioalkyle en chaîne droite comportant 1 à 20 atome(s) de C ou un groupe alkyle, alcoxy ou thioalkyle ramifié ou cyclique comportant 3 à 20 atomes de C ou un groupe alkényle ou alkynyle comportant 2 à 20 atomes de C, où les groupes mentionnés ci avant peuvent chacun être substitués par un radical ou plusieurs radicaux R³ et où un ou plusieurs groupe(s) CH₂ adjacents ou non adjacents dans les groupes mentionnés ci avant peut/peuvent être remplacé(s) par -R³C=CR³-, -C≡C-, Si(R³)₂, Ge(R³)₂, Sn(R³)₂, C=O, C=S, C=Se, C=NR³, -C(=O)O-, -C(=O)NR³-, NR³, P(=O)(R³), -O-, -S-, SO ou SO₂ et où un ou plusieurs atome(s) de H dans les groupes mentionnés ci avant peut/peuvent être remplacé(s) par D, F, CI, Br, 1, CN ou NO₂, ou un système de cycle aromatique ou hétéroaromatique comportant 5 à 60 atomes de cycle aromatique, lequel peut dans chaque cas être substitué par un radical ou plusieurs radicaux R³, ou un groupe aryloxy ou hétéroaryloxy comportant 5 à 60 atomes de cycle aromatique, lequel peut être substitué par un radical ou plusieurs radicaux R³, où deux radicaux R² ou plus peuvent être liés l'un à l'autre ou les uns aux autres et peuvent former un cycle ou un système de cycle ;
R³ est, pour chaque occurrence, de manière identique ou différente, H, D, F, CI, Br, 1, B(OR⁴)₂, CHO, C(=O)R⁴, CR⁴=C(R⁴)₂, CN, C(=O)OR⁴, C(=O)N(R⁴)₂, Si(R⁴)₃, N(R⁴)₂, NO₂, P(=O)(R⁴)₂, OSO₂R⁴, OR⁴, S(=O)R⁴, S(=O)₂R⁴, un groupe alkyle, alcoxy ou thioalkyle en chaîne droite comportant 1 à 20 atome(s) de C ou un groupe alkyle, alcoxy ou thioalkyle ramifié ou cyclique comportant 3 à 20 atomes de C ou un groupe alkényle ou alkynyle comportant 2 à 20 atomes de C, où les groupes mentionnés ci avant peuvent chacun être substitués par un radical ou plusieurs radicaux R⁴ et où un ou plusieurs groupe(s) CH₂ adjacents ou non adjacents dans les groupes mentionnés ci avant peut/peuvent être remplacé(s) par -R⁴C=CR⁴-, -C≡C-, Si(R⁴)₂, Ge(R⁴)₂, Sn(R⁴)₂, C=O, C=S, C=Se, C=NR⁴, -C(=O)O-, -C(=O)NR⁴-, NR⁴, P(=O)(R⁴), -O-, -S-, SO ou SO₂ et où un ou plusieurs atome(s) de H dans les groupes mentionnés ci avant peut/peuvent être remplacé(s) par D, F, Cl, Br, I, CN ou NO₂, ou un système de cycle aromatique ou hétéroaromatique comportant 5 à 60 atomes de cycle aromatique, lequel peut dans chaque cas être substitué par un radical ou plusieurs radicaux R⁴, ou un groupe aryloxy ou hétéroaryloxy comportant 5 à 60 atomes de cycle aromatique, lequel peut être substitué par un radical ou plusieurs radicaux R⁴, où deux radicaux R³ ou plus peuvent être liés l'un à l'autre ou les uns aux autres et peuvent former un cycle ou un système de cycle ;
R⁴ est, de manière identique ou différente pour chaque occurrence, H, D, F ou un radical organique aliphatique, aromatique et/ou hétéroaromatique comportant 1 à 20 atome(s) de C, où, en outre, un ou plusieurs atome(s) de H peut/peuvent être remplacé(s) par D ou F ; deux substituants R⁴ ou plus peuvent ici également être liés l'un à l'autre ou les uns aux autres et former un cycle ou un système de cycle ;
Ar¹ est, pour chaque occurrence, de manière identique ou différente, un système de cycle aromatique ou hétéroaromatique comportant 5 à 30 atomes de cycle aromatique, lequel peut être substitué par un radical ou plusieurs radicaux R³;
ou un polymère, un oligomère ou un dendrimère contenant un ou plusieurs composé(s) de la formule (I), (II) ou (III), où la/les liaison(s) sur le polymère, l'oligomère ou le dendrimère peut/peuvent être localisée(s) au niveau de n'importe quelles positions souhaitées dans la formule (I), (II) ou (III) qui sont substituées par R¹ ou R², est utilisé en tant que matériau de transport de trous dans une couche de transport de trous ou dans une couche d'injection de trous et/ou est utilisé en tant que matériau de matrice dans une couche d'émission et/ou est utilisé en tant que matériau de transport d'électrons dans une couche de transport d'électrons.

2. Dispositif électroluminescent organique selon la revendication 1, **caractérisé en ce que** 0, 1 ou 2 groupe(s) Z par cycle aromatique à six éléments d'un composé de la formule (I), (II) ou (III) est/sont égal/égaux à N, et les groupes Z restants sont égaux à CR².

3. Dispositif électroluminescent organique selon la revendication 1 ou 2, **caractérisé en ce que** X est choisi parmi C=O, NR¹, O, S, S=O et S(=O)₂.

4. Dispositif électroluminescent organique selon une ou plusieurs des revendications 1 à 3, **caractérisé en ce que** Y est choisi parmi C(R²)₂, NR¹, O et S.

5. Dispositif électroluminescent organique selon une ou plusieurs des revendications 1 à 4, **caractérisé en ce que** R² est choisi pour chaque occurrence, de manière identique ou différente, parmi H, D, F, CN, Si(R³)₃, N(R³)₂ ou un groupe alkyle ou alcoxy en chaîne droite comportant 1 à 20 atome(s) de C ou un groupe alkyle ou alcoxy ramifié ou cyclique comportant 3 à 20 atomes de C, où les groupes mentionnés ci avant peuvent chacun être substitués par un radical ou plusieurs radicaux R³ et où un ou plusieurs groupe(s) CH₂ adjacents ou non adjacents dans les groupes mentionnés ci avant peut/peuvent être remplacé(s) par -C=C-, -R³C=CR³-, Si(R³)₂, C=O, C=NR³, -NR³-, -O-, -S-, -C(=O)O- ou -C(=O)NR³-, ou un système de cycle aromatique ou hétéroaromatique comportant 5 à 20 atomes de cycle aromatique, lequel peut dans chaque cas être substitué par un radical ou plusieurs radicaux R³.

6. Dispositif électroluminescent organique selon une ou plusieurs des revendications 1 à 5, **caractérisé en ce qu'**au moins un groupe R¹ ou R² est présent, lequel est choisi parmi :
- des groupes hétéroaryle comportant 1 à 20 atome(s) de C, lesquels peuvent en option être liés via un ou plusieurs groupe(s) aryle divalent(s) liés entre et lesquels peuvent être substitués par un radical ou plusieurs radicaux R³,
- des systèmes de cycle aromatique ou hétéroaromatique comportant 5 à 20 atomes de cycle aromatique, lesquels peuvent être substitués par un radical ou plusieurs radicaux R³, et
- des groupes arylamine, lesquels peuvent être substitués par un radical ou plusieurs radicaux R³.

7. Dispositif électroluminescent organique selon la revendication 6, **caractérisé en ce que** les groupes hétéroaryle sont choisis parmi pyridine, pyrimidine, pyridazine, pyrazine, triazine et benzimidazole, dont chacun peut être substitué par un radical ou plusieurs radicaux R³, et les systèmes de cycle aromatique ou hétéroaromatique sont choisis parmi naphtyle, anthracényle, phénanthrényle, benzanthracényle, pyrényle, biphényle, terphényle et quaterphényle, dont chacun peut être substitué par un radical ou plusieurs radicaux R³.

8. Dispositif électroluminescent organique selon une ou plusieurs des revendications 1 à 7, **caractérisé en ce que** R¹ est choisi pour chaque occurrence, de manière identique ou différente, parmi un système de cycle aromatique ou hétéroaromatique comportant 5 à 30 atomes de cycle aromatique, lequel peut être substitué par un radical ou plusieurs radicaux R³.

9. Dispositif électroluminescent organique selon une ou plusieurs des revendications 1 à 8, **caractérisé en ce que** R³ est choisi pour chaque occurrence, de manière identique ou différente, parmi H, D, F, CN, Si(R⁴)₃, N(R⁴)₂ ou un groupe alkyle ou alcoxy en chaîne droite comportant 1 à 20 atome(s) de C ou un groupe alkyle ou alcoxy ramifié ou cyclique comportant 3 à 20 atomes de C, où les groupes mentionnés ci avant peuvent chacun être substitués par un radical ou plusieurs radicaux R⁴ et où un ou plusieurs groupe(s) CH₂ adjacents ou non adjacents dans les groupes mentionnés ci avant peut/peuvent être remplacé(s) par -C=C-, -R⁴C=C_{R}⁴-, Si(R⁴)₂, C=O, C=NR⁴, -NR⁴-, -O-, -S-, -C(=O)O- ou -C(=O)NR⁴-, ou un système de cycle aromatique ou hétéroaromatique comportant 5 à 20 atomes de cycle aromatique, lequel peut dans chaque cas être substitué par un radical ou plusieurs radicaux R⁴.

10. Dispositif électroluminescent organique selon une ou plusieurs des revendications 1 à 9, **caractérisé en ce que** R⁴ est choisi pour chaque occurrence, de manière identique ou différente, parmi H, D, F, ou un radical organique aliphatique, aromatique et/ou hétéroaromatique comportant 1 à 20 atome(s) de C, où, en outre, un ou plusieurs atome(s) de H peut/peuvent être remplacé(s) par D ou F.
